# EUROPEAN PATENT SPECIFICATION

(11) **EP 3 200 777 B1**
(45) Date of publication and mention of the grant of the patent: **19.06.2019**
(21) Application number: 15845907.3
(22) Date of filing: 28.09.2015
(51) Int. Cl.: A61K 31/12, A61K 31/465, A61K 9/00, A61K 9/06, A61K 9/68, A61P 35/00

(54) **MICROSIZED CURCUMIN COMPOSITIONS**
CURCUMINZUSAMMENSETZUNGEN IN MIKROGRÖSSE
COMPOSITIONS DE CURCUMINE À PARTICULES DE L'ORDRE DU MICRON

(30) Priority: 29.09.2014 US 201461996977 P; 21.01.2015 US 201514602178
(43) Date of publication of application: 09.08.2017
(73) Proprietor: The Board of Supervisors of Louisiana State University & Agricultural & Mechanical College, Baton Rouge, Louisiana 70803 (US)
(72) Inventor: NATHAN, Cherie-Ann, Shreveport, Louisiana 71130 (US)
(74) Representative: HGF Limited
(86) International application number: PCT/US2015/052726
(87) International publication number: WO 2016/053903

(56) References cited:
- EP-A1- 2 711 006
- WO-A1-2011/112386
- US-A1- 2003 084 912
- US-A1- 2008 076 821
- US-A1- 2012 052 095
- US-A1- 2013 029 905
- US-A1- 2013 029 905
- Anonymous: "Archive History for NCT01160302 study on September 9, 2014 Curcumin Biomarker Trial in Head and Neck Cancer", Clinical Trials.gov archive, 9 September 2014 (2014-09-09), pages 1-7, XP055467614, Retrieved from the Internet: URL:https://www.clinicaltrials.gov/ct2/his tory/NCT01160302?V_8=View#StudyPageTop [retrieved on 2018-04-17]
- C. A. CLARK ET AL: "Curcumin Inhibits Carcinogen and Nicotine-Induced Mammalian Target of Rapamycin Pathway Activation in Head and Neck Squamous Cell Carcinoma", CANCER PREVENTION RESEARCH, vol. 3, no. 12, 17 September 2010 (2010-09-17), pages 1586-1595, XP055424059, United States ISSN: 1940-6207, DOI: 10.1158/1940-6207.CAPR-09-0244
- Doddabele Madhavi ET AL: "Bioavailability of a Sustained Release Formulation of Curcumin", Integrative medicine (Encinitas, Calif.), 1 June 2014 (2014-06-01), pages 24-30, XP055467339, United States Retrieved from the Internet: URL:https://www.ncbi.nlm.nih.gov/pmc/artic les/PMC4684130/pdf/24-30.pdf [retrieved on 2018-04-16]
- Schiborr C. et al.: "The oral bioavailability of curcumin from micronized powder and liquid micelles is significantly increased in healthy humans and differs between sexes", Mol. Nutr. Food Res., March 2014 (2014-03), pages 516-527, XP055404740, Retrieved from the Internet: URL:https://onlinelibrary.wiley.com/doi/ep df/10.1002/mnfr.201300724 [retrieved on 2018-04-16]
- DATABASE WPI Week 201369 Thomson Scientific, London, GB; AN 2013-P86381 XP002780142, & CN 103 054 807 A (SUZHOU LEINA DRUG RESEARCH & DEV CO LTD) 24 April 2013 (2013-04-24)
- CLARK, C. A. ET AL.: 'Curcumin inhibits carcinogen and nicotine-induced mammalian target of rapamycin pathway activation in head and neck squamous cell carcinoma' CANCER PREVENTION RESEARCH vol. 3, no. 12, 01 December 2010, pages 1586 - 1595, XP055424059 DOI: 10.1158/1940-6207.CAPR-09-0244

## Description

### BACKGROUND

### Field of the Invention

This invention relates to a pharmaceutical composition for oral transmucosal delivery, comprising microsized curcumin, and a buffer to maintain the pH at 4.5-6.5, wherein the pharmaceutical composition is formed as a lozenge or a gum, and the microsized curcumin are between 100 and 5000 micrometers in size, as well as to said composition for use in treating or preventing a tumor in a patient.

### State of the Art

Annually there are ∼40,000 new head and neck squamous cell cancers (HNSCC) patients and 13,000 deaths in the US and 500,000 new cases worldwide. Despite advances in treatment, the overall survival rates (∼45%) have not improved significantly over the last three decades. Treatment failure in early stage disease is a result of second primary tumors (SPT) and, in advanced stage disease development, of local recurrence and metastasis resulting in morbidity and mortality. As a result of field cancerization, the probability of SPT occurs at a constant risk of 4-7% per year following initial treatment but can be as high as 22% within 5 years with a 5-year survival rate of 25% in these patients. Hence there is a need for chemopreventive agents to delay, arrest or reverse carcinogenesis. The entire mucosa exposed to the carcinogens in tobacco and alcohol have often undergone atypical changes. Moderate to severe dysplasia of the oral cavity and larynx is associated with a well-defined risk of progressing to invasive cancer in 33%-44% of patients. Hence, chemoprevention agents can be used as adjuvant therapy to prevent recurrences in HNSCC which occur in 2-3 years. One agent which appears to have promising potential in prevention of tumor progression is curcumin. Curcumin has been proposed as a chemoprevention agent but has poor bioavailability. Problems with dysphagia or difficulty swallowing occur from the cancer itself and from resection, mucositis, and xerostomia following chemotherapy and/or radiation therapy making it difficult for patients to swallow large pills which would be the case with curcumin as large doses are needed to overcome poor bioavailability.

*Lessons learned from previous studies and the need for a novel agent:* Although preclinical studies have shown >1,000 compounds have chemopreventive properties, very few have made it to clinical trials. Retinoids are the best-studied class with five randomized retinoid trials conducted to date in premalignant oral lesions, that were shown to prevent recurrence and SPT. Although the trials showed significant response rates, dose related mucocutaneous toxicity has been the major adverse effect encountered in these trials. High relapse rates were also noted which indicates the need for prolonged maintenance therapy. In trials with retinoids for HNSCC patients, although SPTs were significantly reduced, there was no change in survival believed to be due to high toxicity-related drop out in the retinoid arm, highlighting the importance of thoroughly investigating dietary supplements such as curcumin for efficacy.

*Improvement of toxicity*: Non-steroidal anti-inflammatory drugs (NSAIDs) have also been widely investigated as chemoprevention agents, due to the anti-angiogenic properties of these agents in rodent models. A large study on the chemopreventive effects of the COX-2 inhibitor Celebrex® was halted because of increased risk of cardiovascular events and toxicities in patients. The COX-2 inhibitor Vioxx® is now removed from the market and the use of the other COX-2 inhibitor, Celebrex®, is markedly limited. There is a focus now to induce tumor cell apoptosis while compensating for COX function to increase efficacy and minimize toxicity. Given the need for a safer agent and the long term use of chemopreventive agents, curcumin has attracted attention due to its antitumor activity and negligible toxicity in humans and animals.

*Curcumin and its current problems:* A natural product isolated from turmeric, curcumin has been implicated as a powerful therapeutic in a variety of human cancers because of its ability to induce apoptosis and is currently undergoing clinical trials for colon, skin, pancreatic, and hematologic cancers, although its effect on HNSCC has been limited for *in vivo* benefit due to low gastrointestinal absorption. Studies have shown increased bioavailability of curcumin when administered in conjunction with the black pepper extract piperine in both rats and humans (2000% in humans with a single dose), although the efficacy remains controversial. The commercially available combination of curcumin and piperine, Curcumin C3 Complex® (Sabinsa Corp. Piscataway, NJ), is currently being evaluated in a phase III trial of metastatic colon cancer, with no dose-limiting toxicity reported in human clinical trials of curcumin up to 10 g/day.

Pharmacologically active levels of curcumin can be achieved in colorectal tissue in patients taking oral curcumin due to prolonged local contact. Although data is lacking, it is unlikely that pharmacologically active concentrations of curcumin can be achieved in tissues that are not directly exposed to a local application. Pharmacokinetic studies of curcumin indicate low bioavailability of curcumin following oral administration. There has been significant controversy with regards to Bioperine® and its role in improving the bioavailability of curcumin. Toxic side effects are inevitable for chemotherapy regimens, but are unacceptable for chemoprevention. Daily dosing of curcumin up to 8 grams daily for four months demonstrated no dose-limiting toxicity, although higher dosing was prohibited due to bulkiness of pills, which would pose a serious problem to head and neck cancer patients post-surgery or during radiation when swallowing is often impaired.

Since curcumin's poor bioavailability and the relatively high concentrations required for efficacy have limited its use, there is a desirability to improve the bioavailability and serum levels of curcumin in patients, especially head and neck cancer patients. Clark et al. describe in Cancer Preservation Research, 3(12), 2010, oral administration of curcumin C3 complex for the treatment of head and neck squamous cell carcinoma (HNSCC). WO-A-2011/112386 discloses a dispersion of a microparticulated water-insoluble compound, e.g. curcumin, in a beverage with pH < 6, preferable 2.5-4.5.

### SUMMARY OF THE INVENTION

The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy.

This invention is directed in part to the discovery that microsized particles (such as microgranules) of curcumin significantly enhances the transmucosal delivery of curcumin when placed in the oral cavity of the mouth. Such an improvement is contraindicated as micronizing poorly soluble drugs is known to increase the water solubility as opposed to increase the mucosal delivery. Without being limited to any theory, it is believed that the large amounts of curcumin in the delivery vehicle allows for gradual release of curcumin from the carrier into the mucosal membrane, thereby increase the systemic concentration of curcumin in the blood stream. As sufficient systemic serum levels of curcumin will down-regulate one or more genes associated with cancer cell proliferation.

Disclosed herein are compositions and methods that allow a sufficient amount of curcumin to contact with the mucous membranes and be absorbed in the oral cavity for the treatment of diseases such as head and neck disorders or disorders of the upper aerodigestive track. It has been surprisingly discovered that delivery of curcumin at least in part through the mucous membranes in a sufficient amount in the form of microparticulates (also called micronsized particles) significantly increases the serum levels of curcumin to a level that is sufficient to down-regulate one or more biomarkers known to be associated with tumor proliferation, such as FGF-2, GRO, MIP-1b, IP-10 and TNFα. In some aspects, the average curcumin serum level provided by the micronsized particles is at least 150 % or 200 % of that provided by oral formulations of curcumin tablets or capsules that do not comprise micronsized particles of curcumin. In some aspects, the average steady state levels of curcumin is at least about 20 ng/mL, at least about 40 ng/mL, at least about 50 ng/mL, at least about 60 ng/mL, at least about 70 ng/mL, at least about 80 ng/mL, or at least about 90 ng/mL.

In one aspect, provided is a formulation comprising microsized particles comprising curcumin or a pharmaceutical composition of such microsized particles, either being suitable for transmucosal delivery. The pharmaceutical composition comprises a pharmaceutically acceptable excipient compatible with transmucosal delivery and a therapeutically effective amount of curcumin wherein said curcumin is present in each dosage form of the composition at an amount of at least about 2 g and no more than about 10 g. In some aspects, the microsized particles or the pharmaceutical composition of such microsized particles further comprise nicotine. In some aspects, the microsized particles or the pharmaceutical compositions of such microsized particles are acidic, such as having a pH of from about 5 to less than 7. In some aspects, the microsized particles or the pharmaceutical compositions of such microsized particles further comprise an pharmaceutically acceptable acid. The invention relates to a pharmaceutical composition for oral transmucosal delivery, comprising microsized curcumin, and a buffer to maintain the pH at 4.5-6.5, wherein the pharmaceutical composition is formed as a lozenge or a gum, and the microsized curcumin are between 100 and 5000 micrometers in size.

In some aspects, the composition comprises microsized particles comprising curcumin. In some aspects, the microsized particles have a size of no more than about 2500 microns, or no more than 2000 microns, such as between about 100 microns and about 2000 microns. In some aspects, the composition comprises microsized particles comprising curcumin. In some aspects, the microsized particles have a number average size of no more than about 2500 microns, or no more than 2000 microns, such as between about 100 microns and about 2000 microns. In some aspects, the microsized particles are administered directed to the mucous membrane, such as the sublingual area of the month. In some aspects, the microsized particles are formulated into a form, such as a chewing gum or capsule (such as dissolvable capsules), that is more conveniently administered transmucosal. In some aspects, the microsized particles are packaged in pouches or capsules that can be opened before administration.

By delivering curcumin, and optionally with other therapeutic agents locally, systemic bioavailability at therapeutic concentrations are achieved. In some aspects, the present invention addresses this problem by providing solid formulations and methods for the delivery of curcumin and other therapeutic agents by absorption through the oral mucosa.

In accordance with one embodiment, this invention provides a solid formulation in the form an excipient facilitate mucosal delivery of curcumin. According to the invention, the excipients provided are lozenges or chewing gum.

In another embodiment, provided are solid formulations containing curcumin and other agents such as phytochemicals and/or vitamins and minerals.

In an embodiment, the invention relates to a therapeutic amount of a composition of claim 1, wherein the amount of said composition is sufficient to provide a serum concentration of curcumin of at least 20 ng/mL for a period of from about 1-2 hours following the initial administration for use in a method for providing a therapeutic blood serum concentration of curcumin in a patient, wherein said method involves oral transmucosal administration.

In another aspect, provided is a method for treating a cancer in a patient in need thereof, which method comprises transmucosally administering a therapeutic amount of a microsized curcumin or a composition thereof to the patient wherein the patient maintains a serum concentration of curcumin of at least 20 ng/mL for about 2 hours.

In another aspect, provided is a method for preventing the formation of a cancer in a patient susceptible to the formation of the cancer, which method comprises transmucosally administering a therapeutic amount of a microsized curcumin or a composition thereof to the patient wherein the patient maintains a serum concentration of curcumin of at least 20 ng/mL for at least about 2 hours. In an embodiment, the invention relates to a composition of claim 1 for use in treating or preventing a tumor in a patient in need thereof by oral transmucosal administration.

These and other embodiments of the invention are further described in the text and claims that follow.

### BRIEF DESCRIPTION OF THE DRAWINGS

Figure 1 represents the plot of growth inhibition of HNCC cells *in vivo* following treatment with the Curcumin C3 Complex®.
Figure 2 is a western blot of tissue samples from sleep apnea non-cancer patients (1-4 and 7-10) and cancer patients (5, 6, 11, and 12) tumor (T) and histologically tumor-free margin (M) probed with antibodies against phospo-Akt (A) or phospho-mTOR (B).
Figure 3 represents curcumin's effects on NFκB and Akt/mTOR pathways in HNSCC cell lines *in vitro* (**A**) and *in vivo* (**B**).

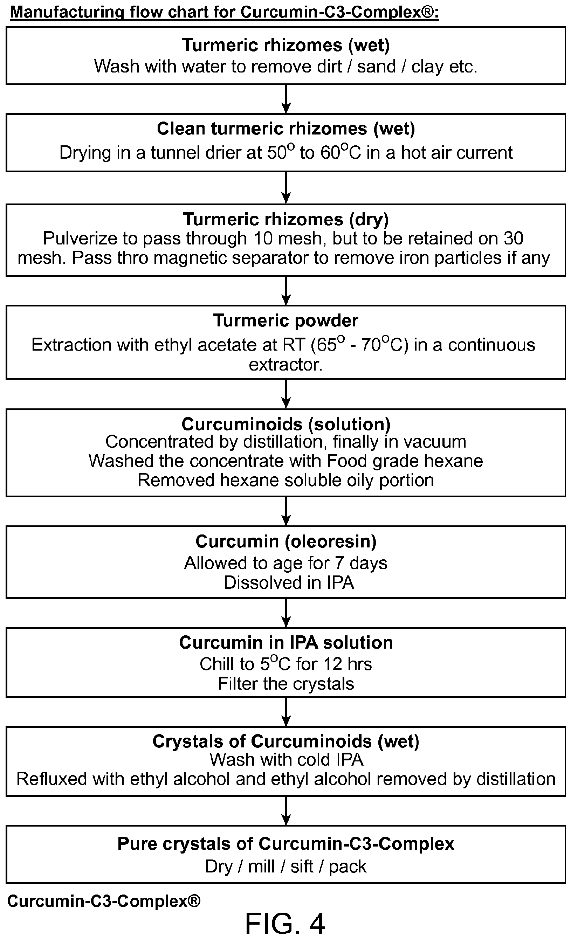

Figure 4 represents a Manufacturing flow chart for an embodiment of curcumin microparticulates Curcumin-C3-Complex®.

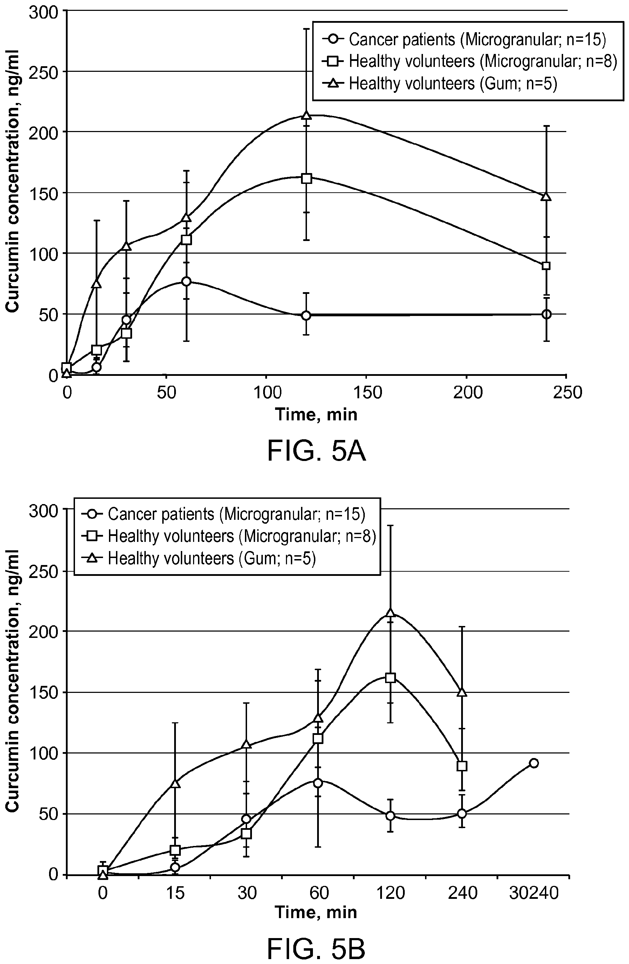

Figure 5A and 5B represent serum levels of curcumin in head and neck cancer patients and healthy volunteers consumed 4 g of microgranular formulation of curcumin once (volunteers) or twice daily (cancer patients). Serum samples were collected at 0, 0.25, 0.5, 1, 2, 4 hours after the initial dose, and for cancer patients only, at 3-4 weeks after daily curcumin consumption. Mean +/- SEM is shown.

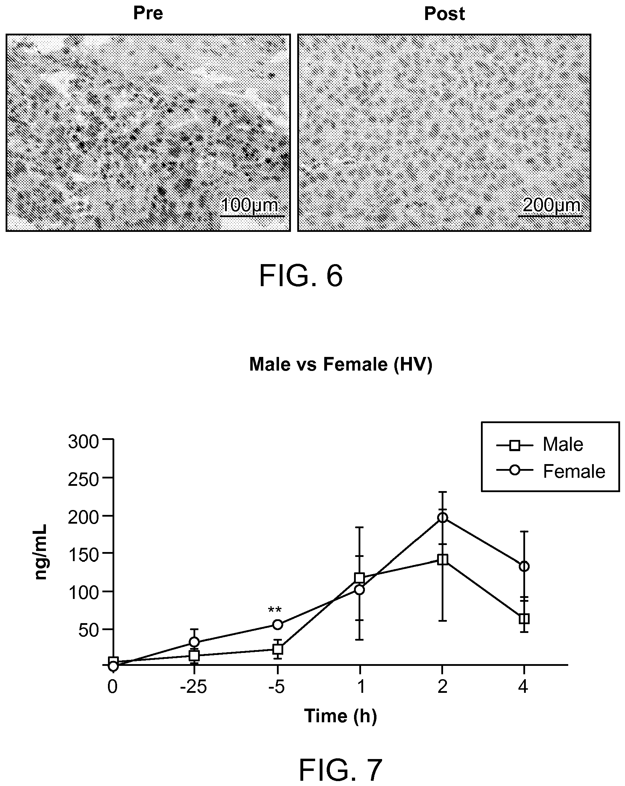

Figure 6 is an analysis of FGF-2 expression by IHC. Representative matched tumor samples collected before (**Pre**; *note strong positive dark staining*) and after consumption of 8 g of curcumin daily for 3-4 weeks (**Post**) are shown.
Figure 7 shows Avg. Serum Curcumin (mean ± SE) of male (5) and female (3) healthy volunteers **Significant at 5% level (p-value = 0.03).

### DETAILED DESCRIPTION OF THE INVENTION

In aspect, provided is a solid oral formulation comprising a therapeutically effective amount of curcumin and a pharmaceutically acceptable carrier for oral mucosal delivery of said curcumin.

It must be noted that, as used in the specification and the appended claims, the singular forms "a," "an" and "the" include plural referents unless the context clearly dictates otherwise. Thus, for example, reference to "a pharmaceutically acceptable carrier" includes mixtures of two or more such pharmaceutically acceptable carriers, and the like.

"Optional" or "optionally" means that the subsequently described event or circumstance can or cannot occur, and that the description includes instances where the event or circumstance occurs and instances where it does not. For example, the phrase "optionally cleaning step" means that the cleaning step may or may not be performed.

The term "comprising" is intended to mean that the compositions and methods include the recited elements, but not excluding others. "Consisting essentially of' when used to define compositions and methods, shall mean excluding other elements of any essential significance to the combination. For example, a composition consisting essentially of the elements as defined herein would not exclude other elements that do not materially affect the basic and novel characteristic(s) of the claimed invention. "Consisting of' shall mean excluding more than trace amount of other ingredients and substantial method steps recited.

The term "about" when used before a numerical value indicates that the value may vary within a reasonable range, such as ± 5%, ± 1%, and ± 0.2% of the stated value.

In one aspect, there is a unit dose curcumin composition suitable for transmucosal delivery which composition comprises microsized curcumin, wherein said microsized curcumin is present in each unit dose at an amount of at least 2 g and no more than 10 g. In some aspects, the unit dose composition comprises about 2 g, 3 g, 4 g, 5 g, 6 g, 7 g, 8 g, 9 g, or about 10 g of curcumin, or any range between any two of the numbers (end point included). In some aspects, the unit dose is delivered once, twice or three times daily.

In one aspect, there is provided a pharmaceutical composition in unit dose suitable for transmucosal delivery which composition comprises a pharmaceutically acceptable excipient compatible with transmucosal delivery and a therapeutically effective amount of curcumin wherein said curcumin is present in each dosage form of the composition at an amount of at least about 2 g and no more than about 10 g. In some aspects, the composition comprises about 2 g, 3 g, 4 g, 5 g, 6 g, 7 g, 8 g, 9 g, or about 10 g of curcumin, or any range between any two of the numbers (end point included). In some aspects, the unit dose is delivered once, twice or three times daily.

In some aspects, the composition comprises microsized particles comprising curcumin. In some aspects, at least 80 %, at least 85 %, at least 90 %, at least 95 % or all of the microsized particles have a size of no more than about 5000 microns, no more than about 4500 microns, 4000 microns, 3500 microns, 3000 microns, 2500 microns, 2000 microns, 1500 microns, 1000 microns, 500 microns, 1000 microns, or 500 microns. In some aspects, at least 80 %, at least 90 %, at least 85 %, at least 95 % or all of the microsized particles have a size of no more than about 5000 microns, and no less than about 500 microns, no less than about 1000 microns, or 1500 microns, or 2000 microns, or 2500 microns, or 3000 microns, or 3500 microns, 4000 microns, such as between about 500 microns and about 5000 microns, between about 500 microns and about 2000 microns, between about 500 microns and about 1500 microns, between about 1000 microns and about 2000 microns, between about 700 microns and about 1500 microns, between about 1000 microns and about 1500 microns, or in a range between any two of the numbers above (end point included). In some aspects, microgranular curcumin refers to microsized particles with coarse surface which have greater surface area than a non-granular material due to the coarse surface.

In some aspects, the amount curcumin in the microsized particles is about 10 % weight/weight (w/w) or more of the microsized particles, such as about 15 %, 20 %, 25 %, 30 %, 35 %, 40 %, 45 %, 50 %, 55 %, 60 %, 65 %, 70 %, 75 %, 80 %, 85%, or any range between any two of the numbers (end point included). In some aspects, the amount curcumin in the microsized particles is at least about 50 %, such as about 75 % to 85 % w/w of the microsized particles. In some aspects, the amount curcumin in the microsized particles is about 80 ± 1 % w/w of the microsized particles.

In some aspects, the composition further comprises bisdemethoxycurcumin and/or demethoxycurcumin. The total amount of curcumin, bisdemethoxycurcumin and demethoxycurcumin is referred to as the total curcuminoids. In some aspects, the bisdemethoxycurcumin in the composition is in an amount of about 2.5 to about 6.5 % w/w, such as about 3 % to about 4 %, about 3.31 % w/w of the total curcuminoids. In some aspects, the demethoxycurcumin in the composition is in an amount of about 15 to about 25 % w/w, such as about 17 % to about 20 %, about 18 % w/w of the total curcuminoids. In some aspects, the curcumin in the composition is in an amount of about 70 to about 80 % w/w, such as about 75 % to about 80 %, about 78 to 79 % w/w of the total curcuminoids.

In some aspects, the amount total curcuminoids in the microsized particles is about 10 % weight/weight (w/w) or more of the microsized particles, such as about 15 %, 20 %, 25 %, 30 %, 35 %, 40 %, 45 %, 50 %, 55 %, 60 %, 65 %, 70 %, 75 %, 80 %, 85%, 90 %, 95%, or no less than 95%, or any range between any two of the numbers (end point included). In some aspects, the total curcuminoids in the microsized particles is at least about 50 %, such as about 75 % to 85 % w/w of the microsized particles. In some aspects, the total curcuminoids in the microsized particles is about 80 ± 1 % w/w of the microsized particles.

According to the invention, the microsized particles or the pharmaceutical composition of such microsized particles comprise a buffer, such as a pharmaceutically acceptable acid, that reduces the pH at or near the mucous membrane to 4.5 to 6.5. In some aspects, the microsized particles or the pharmaceutical composition of such microsized particles are acidic, such as having a pH of about 5, 5.5, 6, or 6.5, or any range between any two of the numbers (end points inclusive). In some aspects, the microsized particles or the pharmaceutical compositions of such microsized particles comprise a pharmaceutically acceptable acid, such as 2,2-dichloroacetic acid, 2-oxoglutaric acid, 4-aminosalicylic acid, acetic acid, adipic acid, ascorbic acid, aspartic acid, benzenesulfonic acid, camphoric acid, camphor-10-sulfonic acid, capric acid (decanoic acid), caproic acid (hexanoic acid), caprylic acid (octanoic acid), carbonic acid, cinnamic acid, citric acid, cyclamic acid, galactaric acid, gentisic acid, glucoheptonic acid, gluconic acid, glucuronic acid, glutamic acid, glutaric acid, glycerophosphoric acid, glycolic acid, hippuric acid, isobutyric acid, lactic acid, lactobionic acid, lauric acid, maleic acid, malic acid, malonic acid, mandelic acid, nicotinic acid, oleic acid, oxalic acid, palmitic acid, pamoic acid, proprionic acid, pyroglutamic acid, salicylic acid, sebacic acid, stearic acid, tartaric acid, and undecylenic acid. Without being limited to any theory, it is contemplated that the weak acidic pH improves curcumin absorption.

In some aspects, the microsized curcumin particles or compositions thereof further comprise one or more pharmaceutically acceptable carriers.

In some aspects, the microsized curcumin particles surprisingly provide improved transmucosal absorption and bioavailability of curcumin without the need for significant swallowing by the patient as compared to compositions such as gums, tablets or capsules, which can be difficult for patients having a cancer or premalignant lesion in the month.

In some aspects, the microsized curcumin particles are sterile. Methods of sterilization are known in the art, such as refluxing in curcumin in a solvent, such as ethanol.

Suitable oral solid formulations according to the invention are in the form of chewing gums or lozenges for administration of curcumin through the oral mucosa. Such solid formulations contain pharmaceutically acceptable carriers relevant for that particular form, and such carriers will be well known to one of skill in the art. The formulations may be chewed, sucked, or placed against the buccal cavity or administered sublingually. The solid formulations may be in any number of shapes. Non-limiting examples include flat sticks or formulations having circular, octagonal, or biconvex shapes.

Administration of curcumin microparticulates through a gum formulation allows for heightened exposure to the drug, since added flavor/chewing quality of gum could make for more palatable dosages with extended dose times.

In some aspects, the amount total curcuminoids or curcumin in the gum is about 10 % weight/weight (w/w) or more of the gum, such as about 15 %, 20 %, 25 %, 30 %, 35 %, 40 %, 45 %, 50 %, 55 %, 60 %, 65 %, 70 %, 75 %, 80 %, 85%, 90 %, 95%, or no less than 95%, or any range between any two of the numbers (end point included). In some aspects, the total curcuminoids or curcumin in the gum is at least about 50 %, such as about 75 % to 85 % w/w of the gum. In some aspects, the total curcuminoids or curcumin in the gum is about 80 ± 1 % w/w of the gum.

Pharmaceutical acceptable carriers include ingredients such as buffers, flavorings, aroma agents, sweeteners, colorants, preservatives such as antioxidants, softeners, thickening agents, emulsifiers, glidants, lubricants, and mixtures thereof.

One or more buffers are used to aid in absorption of curcumin through the oral mucosa. Buffers according to the invention are those capable of decreasing saliva pH to 4.5 to 6.5, to about 5 to 6, or to about 4.5 to 5.5 during administration. Suitable buffers include sodium carbonate, sodium bicarbonate, calcium carbonate, potassium carbonate, potassium bicarbonate, sodium potassium dibasic, sodium potassium tribasic, potassium phosphate dibasic, potassium phosphate tribasic, magnesium hydroxide, potassium hydroxide, aluminum hydroxide, and mixtures thereof. In some aspects, buffers comprise about 0.1 to about 10% or about 0.1 to about 5% of the solid formulation.

Flavorants and aroma agents include essential oils and natural and synthetic compounds that mimic the flavors and aromas of fruits. Flavorants include those having a lemon, orange, lime, spearmint, wintergreen, chocolate, cinnamon, vanilla, coffee, or menthol taste. Suitable flavorant amounts include formulations having from about 0.1 to 0.5% by weight of the flavorant.

Sweeteners may be natural or artificial sweeteners. Examples of sweeteners include sucrose, fructose, mannitol, sorbitol, xylitol, powdered sugar, granulated sugar, molasses, saccharin, acesulfame, aspartame, Nutrasweet, Splenda, and mixtures thereof. In some aspects, sweeteners comprise about 0.001 to about 5% or 10% by weight of the solid formulation. In another aspect the sweetener comprises 0.5% by weight of the formulation.

Suitable colorants include dyes, pigments, and natural food colors approved for use as food additives. In some aspects, the colorants comprise about 0.001% to about 0.05% by weight of the formulation.

The solid formulations may also contain oils and lubricants to aid in the preparation of the formulation, such as to prevent sticking to equipment and molds. In some aspects the oils comprise up to 1% by weight of the formulation.

The curcumin used in the formulations may be prepared synthetically or preferably is isolated from natural sources.

In one embodiment, provided is a chewing gum optionally having a coating layer, wherein said gum comprises gum base, corn syrup, citric acid, glycerin, and a therapeutically effective amount of curcumin and optionally a sweetener.

Processes for preparing gum coatings are well known in the art. The coatings aid in enhancing taste and/or product stability during storage. Suitable coatings include those having a sweetened candy coating.

Gum base is a non-nutritive masticatory substance. It is an inert and insoluble non-nutritive product used as a support for the edible and soluble portion of the chewing gum (sweetener, glucose, flavors etc.). The general description "gum base" used on chewing gum products throughout the world is recognized by The Food Chemicals Codex and most national legislation. Gum base is produced through a blend of raw materials which can be categorized in five classes: 1) elastomers; 2) resins to act as binders and softeners; 3) plasticizers to render the elastomer soft to ensure thorough blending of the gum base; 4) fillers to contribute to the overall texture; and 5) antioxidants to prevent oxidation of the gum base and flavors during shelf life. Suitable gum bases include natural and synthetic gum bases. Synthetic gum bases may contain a mixture of elastomers, plasticizers, fillers, softeners, emulsifiers, and waxes. Examples of gum bases include agar, alginate, arabic gum, carob gum, carrageenan, ghatti gum, guar gum, karaya gum, pectin, tragacanth gum, locust beam gum, gellan gum, and xantham gum. The gum base may comprise 10 to 80% by weight of the chewing gum, excluding the weight of any coating layer.

In some embodiments, the amount of curcumin in each piece of gum is between 500 mg and 5500 mg. In other aspects, the amount of curcumin is 500 mg and 2500 mg.

In other embodiments the gum base is sufficient to maintain the curcumin in the gum matrix until delivery.

In one embodiment, the solid formulation contains sweeteners selected from the group consisting of powdered sugar, granulated sugar, molasses, aspartame, Nutrasweet, and Splenda. In one aspect, the solid formulation contains between 0 and 2 g of sweetener.

In another embodiment, provided is a chewing gum having between 0.05 and 0.5 mL corn syrup for each piece of chewing gum. In some aspects, the chewing gum has 0.18 mL of corn syrup.

In another embodiment, provided is a chewing gum having between 5 mg and 25 mg of citric acid. In some aspects, the chewing gum has 12 mg of citric acid.

In another embodiment, provided is a chewing gum having between 0.01 and 1 mL glycerin. In some aspects, the chewing gum has 0.03 mL of glycerin.

In some aspects, the composition has no more than 5 % or does not comprise nanoparticles such as particles having a size of less than 500 nm or 200 nm. In some aspects, the composition does not comprise polymeric nanoparticles with a hydrophobic core and/or a hydrophilic shell, such as polymeric nanoparticles having a medicament trapped within the hydrophobic core of the nanoparticles. In some aspects, the composition does not comprise polymeric nanoparticles having an average diameter of less than or equal to 50-100 nm, with less than 5% being greater than 200 nm in diameter. In some aspects, the composition does not comprise nanoparticles that are PEGylated at the outer surface. In some aspects, the composition does not comprise nanoparticles that are made from (a) N-isoproylacrylamide; (b) either a water-soluble vinyl compound (to make the particle shell more hydrophilic) or a water-insoluble vinyl derivative (to make the particle core more hydrophobic); and/or (c) acrylic acid.

### Methods of Preparation

In one embodiment, provided is a method for folding therapies into a chewing gum which upon chewing will be slowly released in the oral cavity such that up to 350 mg of the therapeutic can be solubilized (or wet), levigated and folded into a single piece of gum.

In the foregoing method, other phytochemicals, including, but not limited to EGCG (green tea polyphenol epigallocatechin-3-gallate), resveratrol, piperine, genistein, lycopene, lutein, perillyl alcohol, alpha-carotene, saponins, terpeneol, terpene limonoids, hesperetin, silymarin, tangeretin, ferulic acid, sulforaphene, and capsaicin can also be folded into the chewing gum as can other nutritional substances, including, but not limited to vitamins such as vitamin B, vitamin C, vitamin D, and vitamin E and minerals such as iron, calcium and potassium, and other nutritional supplements.

In one aspect, the curcumin or curcuminoids and other phytochemical or nutritional supplement is folded into the chewing gum. In other aspects, the amount of phytochemical is between 1 and 500 mg or is 350 mg.

In another aspect of the disclosure phytochemicals, including, but not limited to curcumin, EGCG, resveratrol, piperine, genistein, lycopene, lutein, perillyl alcohol, alpha-carotene, saponins, terpeneol, terpene limonoids, hesperetin, silymarin, tangeretin, ferulic acid, sulforaphene, and capsaicin can be delivered to the oral cavity in the form of a spray or paste, as can other nutritional substances, including, but not limited to vitamins, such as vitamin B, vitamin C, vitamin D, and vitamin E, minerals, such as iron, calcium and potassium, and other nutritional supplements.

In the foregoing method, the paste would consist of up to the maximally soluble amount of the phytochemical or nutritional substance (to make 100 gm):

| | |
|---|---|
| Polyox® WSR-301 | 5 gm |
| Pectin USP | 2 gm |
| Xanthan Gum | 1 gm |
| Gelatin | 2 gm |
| Acacia NF | 2 gm |
| Carboxymethylcellulose (Medium Viscosity) | 4 gm |
| Vaseline (petroleum jelly) | 84 gm |

Said paste should remain in the oral cavity for at least 10 minutes.

In one embodiment, provided are lozenges containing between 500 and 800 mg of curcumin. The lozenge may also contain one or more sweeteners and contain one or more buffers to facilitate transmucosal curcumin absorption.

In one embodiment, curcumin is present in the solid formulation as a nano-particle. Such particles may be formed according to the general methods disclosed in U.S. Patent No. 5,145,684. In an illustrative example, curcumin is pulverized to nanoparticles in the presence of a surface modifier.

In the another embodiment of this invention, other phytochemicals, including but not limited to, EGCG, resveratrol, piperine, genistein, lycopene, lutein, perillyl alcohol, alpha-carotene, saponins, terpeneol, terpene limonoids, hesperetin, silymarin, tangeretin, ferulic acid, sulforaphene, and capsaicin can be used in the foregoing compositions at therapeutically effective concentrations. The phytochemicals can be used alone or in combinations of two or more, such that the combination does not exceed 25-60% (w/w) by weight of the solid formulation.

In other embodiments, the solid formulations further comprise Polyox® WSR-301, Pectin USP, Xanthan Gum, Gelatin, Acacia NF, Carboxymethylcellulose (Medium Viscosity), Vaseline (petroleum jelly) and curcumin or another phytochemical.

In other embodiments, the solid formulations further comprise Methocel® 2% gel and optionally another phytochemical.

In some aspects, the amount of curcumin or of another phytochemical is from 1 mg to the maximally soluble amount of curcumin or other phytochemical.

In some aspects, provided is a method of preparing microsized curcumin particles. In some aspects, the method comprises:
1. cleaning turmeric rhizomes with water to remove dirt, sand, clay, etc. to obtain cleaned wet turmeric rhizomes;
2. drying the cleaned wet rumeric rhizomes, such as in a tunnel drier at an elevated temperature, such as 50 °C to 60 °C in a hot air to obtained dry turmeric rhizomes;
3. pulverizing the dry turmeric rhizomes by methods known in the art, such as using a pulverizer, and optionally passing through 10 mesh sieves obtain a turmeric powder which are retained by 30 mesh sieves, optionally passing through a magnetic separator to remove iron particles;
4. extracting curcuminoids with a solvent such as ethyl acetate at room temperature to about 60 °C to 70 °C, such as in a continuous extractor;
5. concentrating the extract by e.g., distillation followed by vacuum, washing the concentrate with food grade hexane to remove hexane soluble oily portion, and allowing the resulting product to age, such as for 7 days;
6. dissolving the aged product in a solvent such as isopropyl alcohol, and chilling the solution at a temperature (e.g., about 5 °C) for a period (e.g., about 12 hours) to obtain crystals;
7. washing the crystals with a solvent such as isopropyl alcohol, and refluxing with ethyl alcohol, followed by removing ethyl alcohol (e.g., by distillation); and
8. drying, milling and sifting the crystals by methods known in the art to obtain the microsized curcumin particles and optionally packaging the microsized curcumin particles.

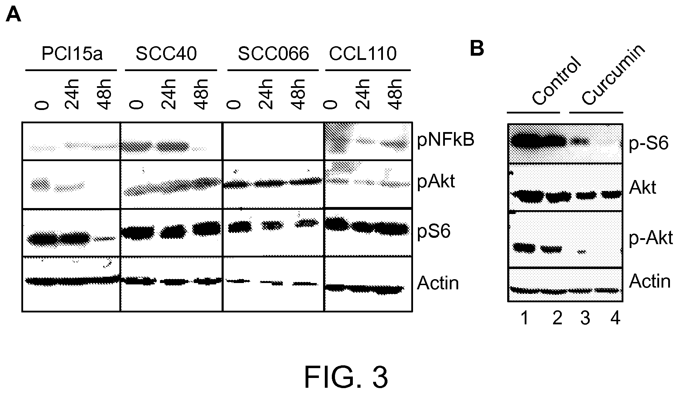

An example of preparing the curcumin microsized particles is shown in Figure 3.

### Methods of Use

In another aspect, provided is a method for maintaining a therapeutic blood serum concentration of curcumin in a subject which method comprises transmucosally administering a therapeutic amount of a microsized curcumin composition to a subject wherein the subject maintains a serum concentration of curcumin of at least 20 ng/mL for a period of at least about 2 hours, or about 4 hours following the initial administration. In some aspects, the serum concentration of curcumin during any of the above periods is maintained at at least about 40 ng/mL, at least about 50 ng/mL, at least about 60 ng/mL, at least about 70 ng/mL, at least about 80 ng/mL, or at least about 90 ng/mL. In some aspects, the curcumin serum Cₘₐₓ is at least about 90 ng/mL, at least about 100 ng/mL, at least about 110 ng/mL, at least about 120 ng/mL, or at least about 130 ng/mL.

In some aspects, the microsized curcumin or a composition thereof is administered at a frequency and for a period, such as that described herein, that the serum concentration of curcumin is maintained for at least 2 weeks after the initial administration of the microsized curcumin or composition. In some aspects, the average steady state levels of curcumin serum concentration is at least about 50 ng/mL, at least about 60 ng/mL, at least about 70 ng/mL, at least about 80 ng/mL, or at least about 90 ng/mL. In some aspects, the curcumin serum Cₘₐₓ is at least about 90 ng/mL, at least about 100 ng/mL, at least about 110 ng/mL, at least about 120 ng/mL, or at least about 130 ng/mL. In some aspects, the serum concentration of curcumin is maintained up to at least 1 week or at least 2 weeks after administration of the microsized curcumin composition. Such a high serum level has not been achieved by administration of curcumin in oral forms such as tablets or capsules known in the art.

In another aspect, the amount of a microsized curcumin composition administered is about 3-5 grams per dose once or twice a day for at least three weeks. In another aspect, the amount of a microsized curcumin composition administered is about 4 grams per dose once or twice a day for at least three weeks. In another aspect, the amount of a microsized curcumin composition administered is about 4 grams per dose once or twice a day for at least four weeks. In some aspects, the microsized curcumin or a composition thereof is administered once, twice or three times daily, and for at least one week, two weeks, three week, one month, two months, six months or one year.

In another aspect, the invention features the use of the foregoing composition(s) to treat head and neck cancers. The foregoing composition(s) can also be used to treat or prevent other problems of the head and neck and upper aerodigestive disorders.

In still another aspect of the disclosure, the foregoing compositions can be used as a prophylactic to deliver nutritional supplements. In one embodiment of the invention, vitamins and/or minerals can be folded into the chewing gum such that the daily requirement of the vitamins and/or minerals is achieved in a patient by chewing one or more pieces of gum each day.

Thus in another aspect, provided is a method for treating a cancer, such as a head and neck cancer or a cancer in the mouth, in a patient in need thereof, which method comprises transmucosally administering a therapeutic amount of a microsized curcumin particles or a composition thereof to the patient wherein the patient maintains a serum concentration of curcumin of at least 20 ng/mL for a period of from about 12 hours following the initial administration and up to at least 2 weeks thereafter.

In another aspect, provided is a chemopreventative method for preventing the formation or reformation of a cancer, such as a head and neck cancer or a cancer in the mouth, in a patient susceptible to the formation or reformation of the cancer, which method comprises transmucosally administering a therapeutic amount of a microsized curcumin or a composition thereof to the patient wherein the patient maintains a serum concentration of curcumin of at least 20 ng/mL for a period of from about 12 hours following the initial administration and up to at least 2 weeks thereafter. In some aspects, the patient that is susceptible to the formation of the cancer is a patient having a premalignant lesion, such as a premalignant oral cavity lesion, including leukoplakia, erythroplakia, palatal lesion of reverse cigar smoking, oral lichen planus, oral submucous fibrosis, discoid lupus erythematosus, and hereditary disorders such as dyskeratosis congenital and epidermolysis bullosa. In some aspects, the patient that is susceptible to the formation of the cancer is a patient who uses one or more tobacco products, such as chewing tobacco, dipping tobacco, taking dissolvable tobacco, smoking cigars or cigarettes, etc. In some aspects, the patient uses the tobacco product regularly, such as at least once a day, and/or in a long term, such as at least a month or a year. In some aspects, the patient that is susceptible to the reformation of the cancer is a patient having a cancer completely or partially cured by any therapy such as surgery, chemotherapy, radiation or prior curcumin therapy, or a combination thereof.

By "chemopreventative" is meant a compound that is capable of preventing the growth or re-growth of a tumor.

By "chewing gum" is meant a sweetened and flavored preparation for chewing.

By "formulation" is meant adding the therapeutic or chemopreventive compound to an acceptable carrier to facilitate delivery of the compound.

By "local delivery" or "local application" is meant delivering a therapeutically effective amount of the drug at the site of the disease, problem or disorder.

By "nutritional intervention" is meant use of a dietary supplement for improving health and reducing the risk of chronic disease or condition.

By "subject" or "patient" is meant a mammal such as a human. In some aspects, a patient is diagnosed of having a disease described herein. In some aspects, a patient is known to have or suspected of having a risk of developing a disease described herein.

By "phytochemical" is meant non-nutritive plant chemicals that have protective or disease preventive properties.

By "proliferative disease" is meant a disease that is caused by or results in inappropriately high levels of cell division, inappropriately low levels of apoptosis, or both. For example, cancers such as lymphoma, leukemia, melanoma, ovarian cancer, breast cancer, pancreatic cancer, bladder cancer, gastric cancer, salivary gland carcinoma, head and neck cancer and lung cancer are all examples of proliferative disease. A myeloproliferative disease is another example of a proliferative disease.

By "therapeutically effective amount" or "therapeutic amount" is meant an amount of a compound sufficient to produce a preventative, healing, curative, stabilizing, or ameliorative effect in the treatment of a condition, e.g., a proliferative disease.

By "treating" is meant the medical management of a subject, e.g. an animal or human, with the intent that a prevention, cure, stabilization, or amelioration of the symptoms or condition will result. This term includes active treatment, that is, treatment directed specifically toward improvement of the disorder; palliative treatment, that is, treatment designed for the relief of symptoms rather than the curing of the disorder; preventive treatment, that is, treatment directed to prevention of disorder; and supportive treatment, that is, treatment employed to supplement another specific therapy directed toward the improvement of the disorder. The term "treatment" also includes symptomatic treatment, that is, treatment directed toward constitutional symptoms of the disorder. "Treating" a condition with the compounds of the invention involves administering such a compound, alone or in combination and by any appropriate means, to an animal, cell, lysate or extract derived from a cell, or a molecule derived from a cell.

Head and neck cancer patients carry a higher risk of developing second primaries, regardless of their initial treatment modality. The occurrence of a subsequent lesion in the entire respiratory and upper aerodigestive tract (UADT) is an important factor for mortality in HNSCC. An agent that can be used to treat HNSCC could be used for other UADT malignancies such as lung and esophagus. For example, squamous cell carcinoma (SCC) is a serious problem due to a prevalence of oral tobacco use and alcohol consumption, especially in the minority population.

Previous studies evaluating the effect of curcumin on TPA-induced (12-0-tetradecanoylphorbol-13-acetate) tumor promotion on mouse skin demonstrated that topical application of 10 µmol curcumin twice weekly inhibited the number of tumors per mouse by 98% (Huang M, Smart R, et al., 1988; Inhibitory effect of curcumin, chlorogenic acid, caffeic acid, and ferulic acid on tumor promotion in mouse skin by 12-O-tetradecanoylphorbol-13-acetate; Cancer Res 48(21):5941-6). Although tumors that develop in the hamster cheek pouch do not histologically or grossly resemble human oral carcinoma, topical application of 10 µmol curcumin to the cheek pouch of hamsters reduced the number of DMBA-induced (7,12-dimethylbenz(a)anthracene) oral tumors by ∼40% when 10 µmol curcumin was applied topically 3 times/week for 24 weeks (Li N, Chen X, et al. 2002; Inhibition of 7,12-dimethylbenz[a]anthracene (DMBA)-induced oral carcinogenesis in hamsters by tea and curcumin; Carcinogenesis 23(8):1307-13). In a more recent study (Li M, Zhang Z, et al. 2007; Curcumin, a dietary component, has anticancer, chemosensitization, and radiosensitization effects by down-regulating the MDM2 oncogene through the PI3K/mTOR/ETS2 pathway; Cancer Res 67(5):1988-96), when curcumin was given by *p.o.* gavage at doses of 5mg/day, 5 days/week for 4 weeks to tumor-bearing nude mice, curcumin inhibited growth of prostate cancer xenograft by ∼50%. To date, a variety of animal models have addressed the chemopreventive properties of curcumin in colon or skin cancer.

### HNSCC cell lines screened for sensitivity to curcumin

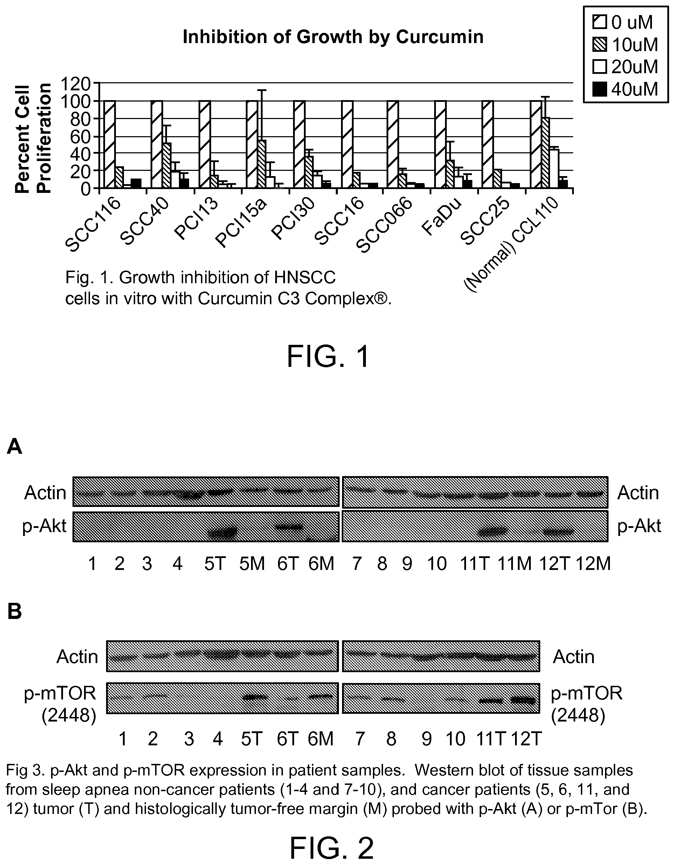

Our *in vitro* data with a variety of HNSCC cell lines has consistently demonstrated sensitivity of SCC cells to curcumin with consistent inhibition of cell proliferation at less than 10 µM curcumin while a higher dose of 20-40 µM was required to see effects in a normal fibroblast cell line CCL110 (Fig. 1). These results suggest the possibility of a favorable therapeutic window *in vivo.* It is striking that a variety of cell lines display a wide range of sensitivity to mTOR inhibitors, including rapamycin (sirolimus) and RAD001 (everolimus), whereas a variety of cell lines are consistently sensitive to curcumin within a narrow range at physiologically relevant concentrations.

### Akt/mTOR pathway is activated in HNSCC and margins showing precancerous changes

Surgical excision of cancer with a curative intent is guided by obtaining histologically tumor-free margins. Most markers lack the sensitivity and ease of applicability for consistent clinical use. In a prospective study on surgical margins of HNSCC patients, we identified eIF4E as the first marker that is elevated in almost all tumor samples, which makes it a sensitive and readily detectable tumor marker in mucosal surgical margins. A total of 276 histologically tumor-free, mucosal surgical margins with a broad range of dysplasia (average of 4.25 margins per patient) from 65 patients were analyzed. Expression of eIF4E has also been shown to correlate with progressive cell transformation in the process of tumorigenesis. We further evaluated the potential of eIF4E downstream of the mTOR pathway as a biomarker by evaluating its expression in the oral cavity where chronic inflammation is a common occurrence. We then determined that over-expression of eIF4E in these margins is functionally active via activation of the Akt/mTOR pathway and also showed that molecular inhibitors of this pathway such as CCI-779, a rapamycin analogue, can inhibit tumor formation and improve survival in a model of minimal residual disease. This preferential activation of the Akt/mTOR pathway in our tumor-free margins compared with the tumor itself could be useful in the design of clinical trials with mTOR inhibitors, such as curcumin.

### The Akt/mTOR pathway as biomarkers in carcinogenesis

We have developed significant data to demonstrate that various parts of the Akt/mTOR pathway can serve as adequate biomarkers. Lippman proposed four criteria for biomarkers in tobacco related epithelial carcinogenesis and our data demonstrates that Akt/mTOR meet these requirements. Criteria #1) Biomarker expression in normal tissue should be different from that in high risk tissue. In Figure 2 we present an analysis of cancer (lanes 5, 6, 11, and 12) and non-cancer (patients with sleep apnea) (lanes 1-4 and 7-10) patient samples. The cancer samples include tumors (T) and histologically tumor-free margins (M). No expression of pAkt was seen in mucosa of non-cancer patients while tumors and some margins express pAkt (Fig 2). Similarly phospho-mTOR (p-mTOR) levels were significantly lower in non-cancer patients compared to cancer patients. Criteria #2) Biomarkers can be detected in small tissue specimens. In our studies we have successfully detected by western blots and even by IHC the various Akt/mTOR markers on samples often as small as 5 mm. Criteria #3) Biomarkers can be expressed in a quantity or pattern that can be correlated with the stage of carcinogenesis. We have successfully demonstrated such correlation in our study on precancerous tissue of the head and neck (Nathan C, Franklin S, 1999; Expression of eIF4E during head and neck tumorigenesis: possible role in angiogenesis; Laryngoscope 109(8):1253-8). Criteria #4) Preclinical or early clinical data indicate that the condition represented by a marker can be modulated by study agents. We now also have exciting data indicating modulation of the mTOR pathway markers with an mTOR inhibitor not only in preclinical models but having completed our Phase 2 biomarker exploratory trial with temsirolimus (CCI-779), a rapamycin analogue, we show modulation of these markers in tumors and PBMCs (a potential surrogate marker) of HNSCC patients (Nathan C, Mills G, et al. 2007; An exploratory biomarker trial of an mTOR inhibitor in subjects with newly diagnosed advanced stage HNSCC; Proceedings AACR Supplement 48:42).

### Mechanism of antitumor effects of Curcumin

A preliminary microarray analysis of gene expression profiles was used to characterize mechanisms of inhibition of cell proliferation in HNSCC exposed to 10µM curcumin for 24-48 hours. Expression of many genes was modulated by curcumin, but two prominently affected pathways of interest were Akt/mTOR and NFκB. These results are the impetus to investigate in detail curcumin's effects on expression levels of genes in these two pathways.

The effects of curcumin on modulation of the NFκB and Akt/mTOR pathways were studied in 3 HNSCC and one normal fibroblast (CCL110) cell line after exposure to 10µM of curcumin for 24 and 48 hours (Fig 3A). Note all HNSCC cell lines showed inhibition of cell proliferation at 10µM of curcumin (Fig1). Three HNSCC cell lines were treated with 10µm curcumin for 24 and 48 hours and effects on the NFκB and Akt/mTOR pathway were analyzed. Figure 3 shows that in PCI15a cells, although no change was noted in pNFκB, both pAkt and pS6 downstream of mTOR were down-regulated at 48 hours. On the contrary, SCC40 cells showed down-regulation of pNFκB after 48 hours and no change in the Akt/mTOR pathway. Finally SCC066 did not express pNFκB and did not show any modulation of the Akt/mTOR pathway. The normal fibroblast cell line CCL110 consistently showed no decrease in any of the pathways tested i.e. pNFκB or Akt/mTOR. It is interesting that although modulation of the biomarkers seems so variable in these cell lines all three HNSCC cell lines consistently showed significant growth inhibitory effects with curcumin at 48 hours. When mice with SCC40 xenografts were treated with curcumin there was a significant decrease in pAkt and pS6 (Fig 3B Lanes 3 and 4) although this same cell line in vitro showed no decrease in the phosphorylation status of the Akt/mTOR pathway. The above data indicates: 1) a panel of biomarkers may be needed in nutritional intervention studies with curcumin as it appears to affect multiple pathways; 2) Although growth inhibitory in SCC40 cell culture, curcumin did not appear to down-regulate Akt/mTOR in cell culture, yet down-regulated the pathway in vivo. Hence the need for human clinical trials to validate biomarkers with nutritional intervention of safe compounds such as curcumin is important prior to embarking on large scale nutritional intervention studies with this bioactive food compound.

Given the above preliminary data it appears that although curcumin appears to have growth inhibitory effects on all 10 HNSCC cell lines tested to date, multiple pathways are inhibited. While curcumin can be used to treat these cancers, other compounds that act on these pathways may also be useful in treating these cancers and it should be readily recognized that such compounds could also be delivered by including in the formulations described herein.

### Chemoprevention model updates

Balb/c nu/nu mice were randomized into treatment groups. In this set of experiments, we pre-treated mice with vehicle or higher doses of curcumin (10 or 15 mg) for 4 days prior to injecting tumor cells (Day 0). Tumors were measured over approximately three weeks. There was a statistically significant difference in tumor volumes between control (vehicle treated) and the 15 mg treated group of mice (p=0.007) by mixed [repeated] ANOVA analysis. However no significant difference was noted at 10 mg of curcumin indicating the importance that high doses may be required to overcome bioavailability. It was previously shown that curcumin has growth inhibitory effects in nude mice with established tumors and that the effects of curcumin were significant in the early stage of tumor development and not the later stages indicating curcumin's potential role as a chemopreventive agent or as adjuvant therapy to prevent recurrence. We show here the growth inhibitory effects of curcumin in the early stages of tumor development as expected with a cytostatic agent. This also points to its role as a chemopreventive agent rather than a therapeutic agent.

Accordingly, the disclosure provides methods of preventing and treating head and neck cancer using curcumin as a nutritional intervention or chemopreventative in patients following treatment of the primary tumor. Given the safety profile of curcumin and nutritional substances, such as vitamins, the solid formulations described can also be used as a prophylactic to prevent the occurrences of diseases or problems.

### Formulations of Curcumin

Illustrative examples of curcumin formulations are described below. In addition to the listed ingredients, any flavoring can be added.

In this example, spearmint oil is used as flavoring. To produce a piece of gum in a troche, the following ingredients are used:

| | |
|---|---|
| Curcumin: | 0.3 g |
| Gum Base: | 1.098 gm |
| Powdered Sugar: | 1.645 gm |
| Corn Syrup: | 0.181 mL |
| Glycerin: | 0.03 mL |
| Citric Acid USP monohydrate: | 0.012 g |

The gum is prepared according to the following procedure:
1. Add gum base, citric acid, glycerin and corn syrup in a beaker. In a second beaker bring enough water to boil to allow a double boiler system. Melt the mixture of gum base ingredients until gooey. May use glass beadlet for bottom of second water beaker.
2. Measure amount of curcumin necessary to make required number of troches. Make a well to pour the melted gum base into on a clean pill tile.
3. Add flavor to the melted gum base and reheat if necessary to insure gooey texture. Pour the melted gum base into the center of the well of curcumin well and knead the two ingredients together using principles of geometric dilution.
4. Mix as fast as possible but with substantial kneading. Kneading too little will cause the gum to break up when chewed. Add to troche mold that is sprayed with Pam and standing by.
5. Remove from troche mold after about 20 minutes. May optionally dispense in a pill bottle.

Alternatively, it may be possible to use glycerin in step 2 and "wet" the curcumin to allow for increased dosage and better mixing.

The following is an example of a lozenge containing curcumin:

| | |
|---|---|
| Curcumin: | 0.3 g |
| Mannitol: | 0.2 g |
| Xylitol: | 1.3 g |
| Sodium carbonate: | 0.5 g |
| Sodium bicarbonate: | 0.15 g |
| Magnesium stearate: | 0.01 g |
| Hydrogenated vegetable oil: | 0.025 g |

The curcumin in dispersed in mannitol and the powders are mixed. The mixture is next compressed using a tablet press.

In another embodiment, the curcumin gum formulations are made as 50 curcumin gum with powdered tangerine flavor 200 mg according to the following formula instructions:

| | |
|---|---|
| curcumin powder 95 % | 10 grams |
| gum, base | 33 grams |
| corn syrup liquid | 9.05 mL |
| glycerin liquid | 5 mL |
| citric acid usp monohydrate | 0.6 grams |
| flavor, tangerine powder | 4.43 grams |
| glycerin liquid | 1.56 mL |
| stevia concentrate 250mg/ml solution | 1.5 mL |
| sodium saccharin powder | 0.1335 grams |

1. Measure curcumin necessary to make required number of troches. Place curcumin on glass pill tile and add powdered flavor and wet with larger quantity of glycerin (#4) to make a smooth paste with half of the curcumin. Leave the remaining curcumin powder intact and make a well with the paste in the middle.
2. Add gum base, citric acid, sodium saccharin powder and the lesser quantity of glycerin (#7) and corn syrup in a beaker.
3. Melt the above mixture of gum base ingredients in microwave until liquified. ("thick maple syrup texture").
4. Add stevia concentrate to the melted gum base and reheat if necessary to insure gooey texture. Stir well with a glass rod that has been sprayed with pam. Pour the melted gum base in the center of the curcumin on the pill tile and knead the two ingredients together.
5. Using the remaining curcumin powder dip gloved fingers into powder to coat them. Mix as fast as possible but with substantial kneading. Kneading too little will cause the gum to break up when chewed. Knead mixture for at least 15 minutes.
6. Add to troche mold that is sprayed with pam. Cover rolling pin with powdered sugar and roll the top of the troche mold to ensure smooth distribution. Turn mold upside down and roll bottom of mold. Remove from troche mold after about 20 minutes. This should complete the process. May dispense in pill bottle.
   *Average weight of each piece of Gum= 1.323 grams

### Additional Compounds

While curcumin has been shown to be effective on its own in treating head and neck cancer and a variety of other disorders, phytochemicals or other therapies with different mechanisms of action, when combined with curcumin could result in even more robust effects. For example, curcumin is known to act on the Akt/mTOR pathway as described herein, whereas another phytochemical, EGCG, has been demonstrated to act at least in part on the c-met signaling pathway. Thus, the combination of these two compounds may result in more efficacious treatments.

In some aspects, the compositions described herein further comprise nicotine. In some embodiments, a unit dosage of the compositions described herein comprises about 0.01 mg to about 4 mg of nicotine or a salt thereof, such as about 0.01 mg, 0.1 mg, 0.5 mg, 1 mg, 2 mg or 4 mg, or any range between any two of the numbers (end points inclusive). In some embodiments, the compositions comprising curcumin and nicotine help patients who are addictive to nicotine containing substances, such as tobacco products, to reduce or cease use of the tobacco products. In some embodiments, the patient can be administered compositions comprising gradually reduced amounts of nicotine based on the patient's reduction in nicotine addiction. For example, the patient is administered a composition comprising a larger amount of nicotine, such as 2 mg to 4 mg, in the beginning of the treatment, and is administered a composition comprising a smaller amount of nicotine, such as 1 mg or 0.5 mg, when the patient's addiction is reduced. In some embodiments, the patient is administered a curcumin composition described herein that contains no nicotine once the addiction is eliminated.

### Detection of Curcumin

In another aspect of the disclosure, provided are methods of detecting curcumin in a sample comprising or suspected of comprising curcumin, which method comprises adding an acid, such as ascorbic acid, to the sample such that the pH of the sample is about 4 to 5, e.g., about 4.6. In some aspects, the method further comprising keeping the sample in the dark or under yellow-filtered light conditions. In some aspects, the sample does not contact with a plastic material. In some aspects, the method further comprises extracting the sample with an organic solvent, such as a solvent comprising ethyl acetate. In some aspects, the method further comprises determining curcumin concentration by reverse-phase high performance liquid chromatography (RP-HPLC). In some aspects, the RP-HPLC is performed suing a solvent gradient comprising varied percentages of 0.1 % glacial acetic acid aqueous solution (Mobile A) and 100 % methanol (Mobile B) as mobile phases, such as 40 % of Mobile A / 60 % of Mobile B over 5 minutes, 35% of Mobile A / 65% of Mobile B for 5-15 minutes, 25% of Mobile A / 75% of Mobile B for 15-30 minutes, and 100% of Mobile B for 30-35 minutes. In some aspects, the method is suitable for detecting and determining the concentration of curcumin in a sample comprising as low as 9 ng/mL of curcumin.

### Example 1

One theory to explain the high frequency of local recurrence and formation of multiple primaries in head and neck squamous cell cancer (HNSCC) is the field cancerization theory. A large subset of oral squamous cell carcinomas arise in the site of preexisting dysplastic lesions and can take years to progress to invasive cancer. Hence, chemoprevention with curcumin holds promise. Most clinical trials of curcumin have utilized capsular formulations, which demonstrated low systemic bioavailability due to poor gut absorption and rapid metabolism. We present results of two clinical trials using a novel microgranular formulation of curcumin in HNSCC patients and healthy volunteers that allows for transmucosal absorption. 15 cancer patients and 8 healthy volunteers were given 4 g of microgranular curcumin on day 1 and blood samples were drawn at various time points to evaluate transmucosal absorption. Cancer patients continued self-administered dosing at 4 g twice daily for 3-4 weeks. Curcumin levels in serum were measured by HPLC, while biologic activity of curcumin was measured with Multiplex Immunoassay on 13 analytes. Pre- and post-biopsy samples were evaluated by immunohistochemistry (IHC) for biomarkers in the Akt/mTOR pathway. We achieved much higher levels of curcumin in serum compared to trials using capsular formulation. We observed a significant decrease in expression of Fibroblast Growth Factor-2 (FGF-2) in post-biopsy samples and decreased serum levels of FGF-2, Granulocyte macrophage colony-stimulating factor (GM-CSF) and Interleukin (IL-17) (p<0.05). These results suggest transmucosal administration of microgranular curcumin leads to an enhanced curcumin bioavailability that is associated with significant biological effects.

HNSCC develops through a multi-step process of genetic, epigenetic, and metabolic changes resulting from carcinogen exposure. While there are numerous causes of head and neck cancer, including viruses such as HPV and EBV, alcohol and tobacco use are at the top of the list. The mucosa of the oral cavity exposed to the carcinogens in tobacco undergoes precancerous changes that often progress to devastating and debilitating oral cavity cancer. The mortality and morbidity from oral tongue cancer in the young is a significant health problem. Moreover, recurrences and second primary tumors (SPT) are common among HNSCC patients, because the entire mucosa has undergone precancerous changes, a phenomenon known as field cancerization. 30%-45% of patients with dysplasia, i.e. precancerous lesions, progress to invasive cancer which occurs in a multi-step process. One of the molecular changes that occurs during progression of precancer to cancer is a switch to an angiogenic phenotype known to be regulated by cytokines and angiogenic factors, such as basic fibroblast growth factor (FGF-2) and vascular endothelial growth factor (VEGF).

Countless studies have researched thousands of compounds to prevent cancer. However, most of the chemopreventives to date, such as the retinoids and COX-2 inhibitors, have had significant side effects when taken over a prolonged period of time (Lippman 1994). When these agents are discontinued as a result of these side effects, the reversal of clinical effects was observed (Hong 1990). Furthermore, although some trials utilizing natural products have shown much promise, unsuccessful trials (such as that of β-carotene) have highlighted the importance of gaining adequate understanding of underlying mechanisms and pharmacology of novel compounds rather than relying primarily on epidemiological data (Gescher 2001). Hence, there is a need for safe, continuous and effective agents thoroughly tested in clinical trials. Curcumin is a polyphenol compound that is derived from turmeric (*Curcuma longa),* a South Asian spice. Curcumin has been found to have anti-inflammatory, anti-oxidative, and anti-carcinogenic properties (Vareed 2008). However, most current supplements of curcumin are hampered by poor bioavailability. Aside from being poorly absorbed by the gut, curcumin undergoes sulfation and glucuronidation at various tissue sites and is rapidly metabolized to these intermediates in the liver (Anand 2007, Ireson 2002). There have been numerous attempts to improve the bioavailability of the nutraceutical curcumin. In Anand's review article (Anand 2007), five methods were devised to improve the compound's bioavailability. An adjuvant piperine was used to reduce metabolite formation. Liposomal curcumin, curcumin nanoparticles, and structural analogues of curcumin were some of the other attempted modifications. While some of these methods show promise, none revealed conclusive evidence of improving curcumin bioavailability (Anand 2007). To date, clinical trials of curcumin have typically involved oral administration of Curcumin C³ Complex™ capsules manufactured by Sabinsa Co. This is commercially available as a curcuminoid mix of 79.85% curcumin, 17.5% demethoxycurcumin, and 2.65% bisdemethoxycurcumin with serum curcumin/metabolite levels typically non-detectable or near the limit of detection (Dhillon 2008, Garcea 2005, Sharma 2004, Sharma 2001, Garcea 2004). Poor bioavailability has negatively impacted its clinical development as an agent for the treatment of chronic systemic diseases. However, curcumin has maintained its promise in the treatment of colorectal cancer due to its ability to bathe the intestinal mucosa over time and bypass the aforementioned constraints of poor bioavailability (Garcea 2005).
We hypothesized that oral transmucosal administration of curcumin in the form of microgranules that can be pouched in the sublingual area of the mouth will improve the bioavailability of the compound, achieving biologic effects at the dose tested. We conducted a clinical trial and compared the serum levels of microgranular curcumin achieved in our study to historical published studies of the capsular formulation of curcumin C³ Complex™ manufactured by the same company, Sabinsa, to determine whether transmucosal administration enhances its bioavailability. Another goal of the study was to evaluate whether improved bioavailability of curcumin translates into biological activity in patients with HNSCC. To analyze any potential differences between cancer patients and healthy subjects, we also evaluated curcumin pharmacology in a set of healthy volunteers.

### Formulation, Dose, and Clinical Trial Design

The clinical trial was performed at the Feist-Weiller Cancer Center / Louisiana State University Health Sciences Center (Shreveport, Louisiana). The study was approved by the Institutional Review Board of LSU Health Sciences Center in Shreveport. All patients provided written informed consent before participating.

For the cancer patients, exclusion criteria were pregnant or nursing females, known hypersensitivity to curry, subjects on NSAIDS or coagulation therapy, prior cancer therapy within 30 days, concurrent chemotherapy or radiation, severely immunocompromised subjects and subjects with a major illness. The curcumin C³ Complex™ was in the form of small beadlets (microgranules), manufactured for our study by Sabinsa Corporation, CA. Following the standard diagnostic biopsy, patients self-administered a 4 grams (g) dose of C³ Complex™ curcuminoid microgranules and were instructed to hold it in the mouth for 10 minutes to facilitate absorption of the drug. We chose a dose of 4 g as our pilot data with microgranular curcumin showed biologic activity at this dosage. Serum was collected at 15 minutes (min), 30 min, 1 hour (hr), 2 hr, and 4 hr after administration. The final serum collection occurred between days 21-28, after patients completed a 3-4 week regimen of 4 g twice daily dosing of curcumin. Curcumin was administered during the standard work up for staging and treatment planning of the head and neck patients. Treatment (chemotherapy and/or radiation) was never delayed due to participation in the trial.

In addition, nine healthy volunteers were recruited and screened with 8 completing the trial. Eligibility criteria included male and female subjects 18-65 years old with no prior dietary curcumin intake within the past 48 hours prior to dosing. The same exclusion criteria were used. Dosing levels / administration and collection schedule were similar to the cancer patients on day 1 as healthy volunteers received only a one-time dose of 4 g.

All blood samples were processed within 24 hours and stored at 4 °C and protected from light. Serum samples were centrifuged, aliquotted into cryovials and stored at 80 °C until biomarker/pharmacokinetic analyses was performed.

### Pharmacological Analysis

### Preparation of Serum Samples for High-Performance Liquid Chromatography (HPLC) Analysis

Serial dilutions of curcumin from a 1 mM stock of C³ Complex™ in 1 mM ascorbic acid in MeOH (pH 4.6) were used as calibrators to establish a standard curve with a detection limit of 9 ng/mL. The acidic pH of the solvent is critical, since curcumin is unstable at basic pH and undergoes slower degradation under acidic conditions (Sharma 2005). Since most solid tumors exhibit acidic conditions in their core, curcumin in direct contact with tumors may present an ideal absorptive environment (Aggarwal 2009). Addition of ascorbic acid to the solvent, along with sample/standard preparation in the dark or under yellow-filtered light conditions, prevents its degradation (curcumin visibly changes from yellow to red when degraded) (Sharma 2005). All preparations were performed in Axygen 2 mL clear microtubes (Part #: MCT-200-C-S), because curcumin reacts with the plastic present in traditional 1.5 mL Eppendorf tubes.

200 µL of each serum sample (in duplicate) was mixed with 200 µL of β-Glucuronidase that also contains sulfatase activity (catalog #G1512; Sigma-Aldrich) and was allowed to incubate at 37 °C in a water bath in order to convert all conjugates to parent compound. The enzyme concentration of 500 units/50 µL in 0.1 M sodium acetate (pH 5.0) was critical for the methodology as higher concentrations caused interference in detecting curcumin. The efficacy of these enzymes was validated with glucuronide and sulfide metabolites, which underwent ≈100% conversion to their free forms upon incubation with the enzymes. Post incubation, enzymatic reactions were quenched using 1 mM ascorbic acid, pH 4.6 in methanol. Each sample was extracted with 1 mL of 5% methanol/95% ethyl acetate, vortexed, and spun for 30 minutes at 14,000 rpm in a cold room centrifuge. The upper organic phase layer was then removed and placed in separate microcentrifuge tubes. The procedure was repeated twice, and pooled serum extracts were concentrated using a Savant SpeedVac. Samples were reconstituted in 100 µL of 1 mM ascorbic acid/methanol solution, mixed in a sonication bath for 2 minutes, and 75 µL were injected on-column.

### HPLC Parameters

Reverse-phase HPLC (Agilent 1100 series) with a Waters uBondapak C18 10 µm 4.6x250 mm analytical column operated at ambient temperature was used to quantify curcumin in serum. The mobile phase was 0.1 % glacial acetic acid (Mobile A) and 100 % methanol (Mobile B). Extracted serum samples were eluted using a gradient starting at 40 % of Mobile A / 60 % of Mobile B over 5 minutes, then 35% of Mobile A / 65% of Mobile B for 5-15 minutes, 25% of Mobile A / 75% of Mobile B for 15-30 minutes, and finally 100% of Mobile B for 30-35 minutes. The column was cleaned of residual curcumin using an injection of 100 µL of isopropanol in 100% of Mobile B for 23 minutes. With all analyses performed at a 1 mL/min flow rate and fluorescence detection at Ex 420 nm and Em 524 nm, curcumin eluted at about 23 minutes.

### Efficacy Studies

### Immunohistochemical Analysis of Molecular Markers

Formalin-fixed, paraffin-embedded tumor tissue samples from patient biopsies or surgical resections were serially sectioned at 5 microns. Tissue sections were then stained with hematoxylin and eosin stains to confirm the diagnosis. Sections were treated with 1:200 rabbit polyclonal to FGF-2 from Santa Cruz Biotechnology (sc-79); and the following antibodies from Cell Signaling: 1:200 rabbit polyclonal to matrix metalloproteinase-9 (MMP-9; #3852); 1:100 rabbit monoclonal to phospho-S6 (pS6; #4858); 1:100 rabbit monoclonal to phospho-Akt (pAkt; #3787); 1:100 rabbit polyclonal to phospho-mTOR (pmTOR; #2971); 1:300 rabbit monoclonal to phospho-4EBP1 (p4EBP1; #2855). The high-resolution Biogenex streptavidin conjugated detection system (Biogenex, San Ramon, CA) was used according to the manufacturer's instructions. The slides were read and scored by the study pathologist (FA) who was unaware of the clinical details. Staining intensity was scored semi-quantitatively. No staining was scored as [-] (minus), weak or focal staining as [+], intermediate staining as [++], and strong staining as [+++].

### Multiplex Immunoassay Analysis of Serum Cytokines and Growth Factors

As IHC staining is subject to inconsistent results due to tumor heterogeneity and sampling errors, we also analyzed the serum samples using MILLIPLEX MAP Human Cytokine/Chemokine Magnetic Bead multiplex assay for changes in cytokine and chemokine levels after curcumin consumption. The Bio-Plex® 200 system with HTF (high-throughput fluidics) was used to conduct the MILLIPLEX MAP Magnetic Bead Immunoassay. 13 different analytes were measured: FGF-2, Granulocyte macrophage colony-stimulating factor (GM-CSF), Interferon g (IFNg), Growth-Related Oncogene (GRO), Interleukin (IL)-13, IL-17, IL-1b, IL-6, IL-8, Inducible Protein (IP)-10, Macrophage inflammatory protein-1β (MIP-1β), Tumor necrosis factor α (TNFα), and VEGF. These analytes were selected based on the results of previous studies showing that these cytokines are potential targets of curcumin. Each standard and serum sample was tested in duplicate.

### Statistical Analysis

Spearman rank correlation analysis was used to correlate curcumin serum levels with decrease in biomarker levels at different time points post curcumin consumption. Paired t-test or the Wilcoxon signed rank test were used to determine significant changes in curcumin levels or biomarker expression at various time points compared to baseline values. Unless otherwise stated, values in the text represent the mean + the SD.

### Characteristics of cancer patients and healthy volunteers.

Demographic and baseline characteristics of cancer patients and healthy volunteers are shown in Tables 1 and 2, correspondingly. 33 Cancer patients were enrolled, 18 were withdrawn and 15 completed the trial. 18 Patients were withdrawn either voluntarily or by the PI due to the following reasons: primary treatment was initiated early after patient's enrollment in the curcumin trial; non-compliance with dosing, blood draws or biopsy; baseline blood chemistry eligibility failure; one patient was unable to take curcumin as directed due to tumor size; one patient had diarrhea after taking the first dose of curcumin (see below). Curcumin treatment was well tolerated in most subjects. One subject experienced diarrhea within 24 hours of taking the first dose of curcumin C³ Complex™. At this time the subject notified us of a history of Crohn's disease that had not been active in over a year. The subject was advised to skip a dose and the diarrhea resolved within 24 hours. The PI decided to discontinue curcumin in this patient.

The median age for the 15 cancer patients who completed the trial was 52 years (range, 36-76 years). 14/15 patients were men (Table 1). All but one of the patients had a history of tobacco use and 9/15 reported present or past heavy alcohol use.

**Table 1. Cancer patient characteristics**

| | Number (%) or Mean±SD; Median (Range) |
|---|---|
| Sex: | |
| Male | 14 (93.3%) |
| Female | 1 (6.7%) |

| Race: | |
|---|---|
| Caucasian | 10 (66.7%) |
| African American | 5 (33.3%) |
| Age (years) | 52.7±11.5, 52.0 (36 - 76) |

| Site: | |
|---|---|
| Oral cavity | 3 (20.0%) |
| Oropharynx | 12 (80.0%) |

| HPV status | |
|---|---|
| Positive | 6 (40.0%) |
| Negative | 3 (20.0%) |
| Not determined | 6 (40.0%) |

| Cancer stage: | |
|---|---|
| 1, 2 or 3 | 4 (26.7%) |
| 4 | 11 (73.3%) |

| T-stage | |
|---|---|
| 1 or 2 | 7 (46.7%) |
| 3 or 4 | 8 (53.3%) |

| N-stage | |
|---|---|
| 0 or 1 | 6 (40%) |
| 2 | 9 (60%) |

| M-stage: | |
|---|---|
| 0 | 15 (100%) |

The median age of healthy volunteers was 42.5 years (range, 22-63 years). Among healthy volunteers 5 out of 8 were men (Table 2). A history of tobacco and alcohol use was not collected on healthy.

**Table 2. Characteristics of healthy volunteers**

| | Number (%) or Mean±SD; Median (Range) |
|---|---|
| Sex: | |
| Male | 5 (62.5%) |
| Female | 3 (37.5%) |

| Race: | |
|---|---|
| Caucasian | 6 (75.0%) |
| African American | 2 (25.0%) |
| Age (years) | 41.8±13.4; 42.5 (22 - 63) |

### Curcumin pharmacology

### Cancer Patients

Serum levels of curcumin in both cancer patients and healthy volunteers are shown in Figure 1. A wide range of concentrations was noted. Compared to baseline levels, there was a significant increase in curcumin levels among cancer patients starting at the 30 min time point and thereafter (p < 0.05). On day 1 of the study, the average serum curcumin concentration peaked at 76.51±168.94 ng/mL around 1 hour following intake of the first dose in cancer patients. Curcumin was undetectable in the serum of two patients on day 1 of the study. 1 patient attained maximum concentration of curcumin at 30 min, 3 patients at 1 hour; 4 patients at 2 hours and 5 patients at 4 hours after curcumin ingestion. At the post time point (3-4 weeks after steady daily dosing), the average level of curcumin was 92.88±133.02 ng/mL and the maximum concentration was 403.52 ng/mL. All but one patient had detectable levels of curcumin at the post time point. The majority of patients, 8 of 15, achieved the highest concentration of curcumin at the post time point. The average Cmax was 127.14±186.65 ng/mL (maximum level of curcumin) with a range of 19.09 to 711.29 ng/mL.

### Healthy Volunteers

All healthy volunteers following intake of one 4 g dose attained detectable levels of curcumin before the 4 hour time point. Compared to baseline levels, there was a significant increase in curcumin levels from the 30 min time point and thereafter (p < 0.05). For healthy volunteers, serum concentrations peaked 2 hours after ingestion of curcumin, with an average concentration of 162.04±121.37 ng/mL. The majority of healthy volunteers, 5 out of 8, attained the highest concentration of curcumin at the 2 hour time point. One healthy volunteer attained a maximum concentration of curcumin at the 1 hour time point while for 2 volunteers this occurred at the 4 hour time point. The Cmax was 189.26±130.37 ng/mL with a range of 33.05 to 406.49 ng/mL; 5 healthy volunteers achieved a maximum level of 100 ng/mL of curcumin or greater.

Two cancer patients and one healthy volunteer had detectable levels of curcumin at baseline (9.77, 22.31, and 25.56 ng/mL). This could be due to patients having some form of curcumin in their diet prior to the trial, although this was an exclusion criterion. Many common food substances such as mustard contain curcumin and patients may not have been aware.

### A comparison of curcumin pharmacology for microgranular formulation administered transmucosally to historical published studies of oral capsular formulation

Next we compared serum levels of curcumin after the administration of the microgranular formulation of curcumin to historical controls, i.e. trials where curcumin from the same company was taken in a capsular formulation at approximately the same dose (see Table 3).

**Table 3. Comparison of our findings using microgranular formulation of curcumin with published studies of capsular formulation of curcumin**

| **Published Studies** | **Capsular formulation of curcumin** | **Microgranular formulation of curcumin (*this study*)** |
|---|---|---|
| Sharma Colon Cancer: 3.6g daily with 30 min, 1 hr, 2 hr, 3 hr, 6 hr and 8 hr collection time points (Sharma 2004) | 16.99 ng/mL mean curcumin at 1 hr^{a} | 76.51±168.94 ng/mL and 111.88±129.71 ng/mL mean curcumin (±SD) at 1 hr time point^{a} among HNSCC patients and healthy volunteers, respectively |
| Garcea Metastatic ColonCancer: 3.6 g daily dose for 1 week with collection at 1hr time points. (Garcea 2004) | Curcumin and its metabolites measured separately. Peak areas for all measurements below or near LOD.^{*} | |
| Garcea Colon Cancer Study: 3.6 g daily for 1 week with collection at 1 hr timepoint on day 7. (Garcea 2005) | Free curcumin detected below the LOD and no metabolites detected.^{*} | |
| Dhillon Advanced PancreaticCancer Study: 8 g daily for 8 weeks. (Dhillon 2008) | Conjugated curcumin in plasma measured at steady state = 22-41 ng/mL. | Post (approx. 3 weeks) was 92.88±133.02 ng/mL (Mean±SD)^{b} among HNSCC patients |

| | | |
|---|---|---|
| ^{*}Below the limit of detection (LOD), which was 1 ng/mL. ^{a} Quantified as parent compound plus metabolites. ^{b} Total curcumin at steady state. | | |

In the dose escalation study on the capsular formulation of curcumin C³ Complex™ (Sabinsa Corporation), Lao et al reported that no curcumin was detected in the serum of subjects administered escalating single doses 500, 1000, 2000, 4000 or 8000 mg of the drug (Lao et al, 2006). Several other studies were unable to detect curcumin in serum samples of patients consuming 3.6 g daily of curcumin. Dr. Sharma studied the effects of capsular curcumin on colon cancer patients that were administered oral doses of 3.6 g of curcumin daily. The study yielded average levels (total curcumin = curcumin + metabolites) of 16.99 ng/mL at the 1 hour following curcumin ingestion (Sharma 2001), whereas the average for our microgranular formulation was 76.51±168.94 ng/mL total curcumin at the same time point. Dhillon's study at MD Anderson evaluated the effects of an 8 g daily dose of capsular curcumin in advanced pancreatic cancer patients and reported steady state serum total curcumin levels of 22-41 ng/mL (Dhillon 2008). Our novel microgranular formulation resulted in more than double these values, with average steady state levels of 92.88±133.02 ng/mL.

### Efficacy Studies

To determine the biologic effects of microgranular curcumin and identify potential biomarkers for future chemopreventive studies, we tested the effects of curcumin on tissue biomarkers, as well as on angiogenic and anti-inflammatory cytokines in serum. Tissue biomarkers

Previously, we demonstrated robust anti-proliferative effects of curcumin in a panel of nine HNSCC cell lines which were associated with inhibition of the AKT/mTOR pathway and downregulation of FGF-2 and MMP-9, both biomarkers of angiogenesis and invasion regulated by the AKT/mTOR pathway. Therefore, we chose to evaluate these biomarkers in HNSCC patients enrolled in the trial. Only FGF-2 was significantly decreased in post-treatment tumor samples in 7 out of 11 patients when compared to evaluable matched pre-treatment tumor samples (p = 0.0261; Figure 2).

### Serum cytokines and growth factors

As IHC staining is subject to inconsistent results due to tumor heterogeneity and sampling errors, we also analyzed serum for changes in cytokine and growth factor levels, comparing baseline to various time points after curcumin. We employed the Milliplex_{MAP} Human Cytokine/Chemokine Magnetic Bead multiplex assay. There was a significant effect of curcumin on the serum level of FGF-2 at time points 4 h (p=0.0078) and a trend towards decreased levels of FGF-2 at 15 min (p=0.084), 30 min (p=0.083) and 1 h (p=0.084) after curcumin administration. Curcumin caused a significant decrease in the serum levels of GM-CSF between 15 min and 2 h (p<0.05). In addition, we detected a significant decrease of serum levels of IL-17 at 1 h (p=0.0342) post curcumin consumption. Curcumin treatment also caused a noticeable decrease in the levels of IFNγ, IL-13, TNFα and VEGF at various time points and an increase in the level of MIP1β. However, because of data variability, there was no statistically significant difference at the various time points for the aforementioned serum biomarkers.

**Table 4. Baseline serum levels of cytokines and growth factors and responses to curcumin consumption in HNSCC patients ‡**

| **Analytes** | **Serum levels (pg/mL)** | | | | | | |
|---|---|---|---|---|---|---|---|
| | **baseline** | **15 min** | **30 min** | **1 hour** | **2 hours** | **4 hours** | **Post (3-4 wks)** |
| FGF-2 | 90.4±142.6 | 53.4±96.7^{ϕ} | 52.4±101.6^{ϕ} | 57.0±115.5^{ϕ} | 45.7±78.9 | 35.9±75.9* | 40.8±53.7 |
| GM-CSF | 30.7±59.8 | 17.7±48.1* | 10.5±24.8* | 10.2±18.9* | 4.4±3.0* | 7.3±13.6 | 19.4±46.6 |
| IL-17 | 29.4±89.7 | 7.4±13.3 | 6.1±11.2 | 4.5±7.4* | 6.2±12.8 | 4.9±3.9 | 5.3±6.1 |

| | | | | | | | |
|---|---|---|---|---|---|---|---|
| ‡ - No significant changes were observed in the other multiplex measures (data not shown). * - p <0.05. ^{ϕ} - 0.05 < p < 0.10. | | | | | | | |

Curcumin's molecular mechanism of action and primary cellular targets are not clearly defined. There is also the problem posed by its low bioavailability. The literature shows many attempts at alternative formulations to improve curcumin absorption. These include chemical modification to prodrugs, dosing formulations (micelles, liposomes, nanoparticles and phospholipid complexes) and combination with other dietary components such as piperine. None of these attempts have had a significant impact on curcumin's bioavailability (Bisht 2009). The purpose of this study was to investigate the effects of a novel microgranular formulation of curcumin administered via transmucosal route on the drug's bioavailability and efficacy in HNSCC.

In our study we observed a significant increase in serum levels of curcumin at 30 min after ingestion of curcumin in both cancer patients and healthy volunteers. When we compared the results of our study with microgranular curcumin administered transmucosally to historical controls, i.e. trials where curcumin from the same company was taken in a capsular formulation at approximately the same dose, our patient serum levels showed appreciable increases in curcumin/metabolite levels relative to previously reported results. Using this formulation to deliver curcumin to patients via prolonged contact with the oral mucosa (transmucosal absorption), given our strong preliminary data, could be an exciting approach to effective and cost friendly biodelivery. Transmucosal administration of microgranular curcumin C³ Complex™ shows a significant improvement in serum concentrations over the same form of the drug swallowed as bulky capsules in other published studies for colon and pancreatic cancer.

Higher plasma levels of curcumin do not necessarily translate to biologic activity. Hence, biomarkers of response were tested to determine if increased curcumin concentrations had biologic effects. Also, identifying a consistent biomarker that can be validated in chemoprevention trials is critical, as the occurrence of cancer as an end point could take years. Furthermore, in future chemoprevention clinical trials of curcumin in subjects with a high risk of developing cancer, no tumors would be available, making serum cytokine analysis an important serum biomarker. We have reported a significant inhibition of FGF-2 expression in HNSCC cells by curcumin (p<0.001) (Sharma 2001). In this study we observed a significant decrease in expression of FGF-2 in post biopsy tumor samples and decreased levels of serum FGF-2, indicating that FGF-2 is potentially an important biomarker for future clinical trials of curcumin. Although many other markers appear promising and trend towards significance, only FGF-2 was consistently affected. It also indicates a potential effect of curcumin in suppressing angiogenesis in the conversion of premalignant to malignant lesions. Importantly in our study of the oral premalignant lesions, we found an upregulation of FGF-2 expression (p<0.001) with increasing grades of dysplasia which correlated with an increase in mean vessel density [Anand 2008]. Acquiring the angiogenic phenotype by premalignant cells is the a critical step in carcinogenesis and FGF-2 is one of the most important angiogenic markers. Oral dysplasias are characterized by deregulations (DNA amplification and homozygous deletion) of multiple components of the FGF signaling network. Furthermore, other serum biomarkers found to be significantly inhibited by curcumin in our study are also known to be implicated in the regulation of angiogenesis and cancer cell invasion. GM-CSF promotes proliferation of endothelial progenitor cells, induces endothelial capillary formation via stimulation of MMP expression and activates JAK2/STAT-3 pathway. IL-17, besides its function as immune marker, is also implicated in stimulating angiogenesis.

We have shown that the problem of poor bioavailability can be overcome by oral transmucosal administration of curcumin that greatly improves systemic bioavailability of the compound. Oral transmucosal administration of curcumin will also obviate the problems posed by swallowing large numbers of bulky pills in patients with head and neck tumors. We demonstrated that improved bioavailability of microgranular curcumin was associated with a significant decrease in FGF-2 and other factors implicated in the regulation of angiogenesis and cell invasion. This shows that curcumin is a potential angiogenic inhibitor in HNSCC and can potentially prevent progression of preneoplastic lesions to invasive cancer. This pilot trial provides the basis for a direct comparison of the microgranular formulation of curcumin and the capsular formulation for patients with oral premalignant lesions.

### Example 2

It is contemplated that the microgranular formulation of curcumin which is safe and efficacious and has improved bioavailability will reverse the process of carcinogenesis in premalignant oral cavity lesions. The primary end point will be change in histologic grade and the size of the lesions after 12 months of treatment. Biopsy results will be correlated with optical images obtained of the lesion prior to the biopsy with the goal of using optical imaging in future studies.

*Study Design:* A multi-institutional placebo-controlled randomized phase 2 chemoprevention trial is conducted using the microgranular formulation described herein.
Enrolled patients with premalignant oral cavity lesions will consume 8 g of microgranular curcumin or placebo daily for a year.

### Schema of the Clinical Trial

1. Registration for the clinical study.
2. Acquisition of preneoplastic tissue, blood and saliva at the time of biopsy of newly diagnosed patients.
3. Treatment with 8 g of curcumin daily for a year.
4. Re-biopsy of tissue in the affected sites and in vivo imaging at 3 and 12 months of study initiation after treatment with curcumin and concurrent in vivo Optical Microscopy by CellVizio for certain patients.
5. Blood collection at 0 hour, 4 hours, 3-4 weeks, 3 and 12 months of the study initiation. 4 hour time point was chosen since a significant decrease of FGF-2 was observed at this time, while the 3-4 week time point was selected as the highest level of curcumin was achieved in the study.
6. Analysis of serum samples for curcumin levels.
7. Analysis of collected serum, saliva and tissue biopsies for biomarkers-molecular targets of curcumin: FGF-2, NF-kB, pSTAT3, COX-2, GM-CSF, IL-17, TNF-α, IL-6, IFNγ and IKKbeta kinase activity in salivary cells. Pre-, on- and post-treatment paraffin-embedded tissue biopsies will undergo IHC staining for H&E and tissue biomarkers (FGF-2, NF-kB-p65, pSTAT3 and COX-2).

Tissue histologic grade will be classified as follows: 1) normal mucosa / benign hyperplasia; 2) low-grade dysplasia; 3) high-grade dysplasia (moderate-severe dysplasia / Carcinoma in situ); 4) invasive cancer. Pathologic complete response will be defined as complete disappearance of dysplasia from the epithelium. Pathologic partial response is an improvement of dysplasia by at least one degree of dysplasia (such as high-grade dysplasia becomes a low- grade dysplasia). No pathologic changes after treatment will be classified as stable disease. Pathologic progressive disease will be defined as worsening of dysplasia (e.g., low-grade dysplasia becomes high-grade dysplasia) or development of invasive cancer during the period of curcumin consumption. In addition the bi-dimensional surface area of the lesions will be recorded and the objective responses will be classified as follows. Complete response will be defined as disappearance of a lesion for at least four weeks. Partial response will be defined as decrease in the cross-sectional area of a lesion by at least 50% for at least four weeks. Stable disease will be defined as a decrease in lesion size of less than 50% in the cross-sectional areas of measurable disease for at least four weeks. Disease progression will be defined as an increase in the cross-sectional areas of lesion(s) by more than 25% or the appearance of a new lesion. Response rates in the curcumin arm will be compared to those in the placebo arm.

The concentrations of curcumin in serum will be determined at the various time points (baseline, 4 hours, 3-4 weeks, 3 and 12 months) and correlated with clinical objective parameters (changes in histological grade and reduction in size). Wilcoxon signed rank test will be used to correlate curcumin level and changes in lesion size and histologic grade. The agreement between in vivo Optical Microscopy by CellVizio and histological examination of tissue biopsies, the current "gold standard" in the assessment of oral precancerous lesions will be examined.

The effect of curcumin on FGF-2 and other key factors in the angiogenic switch in patients' serum and tissue samples will be evaluated. It is contemplated that biomarkers involved in the angiogenic switch of precancer to cancer will be downregulated with the microgranular formulation of curcumin. FGF-2 is a key factor in the angiogenic switch and was found to be the most promising biomarker in our preclinical studies and clinical trials. The effects of curcumin on serum biomarkers of angiogenesis, FGF-2 and other markers such as GM-CSF, IL-17, MMPs, VEGF-A, TGFs, IL-6 will be evaluated using Milliplex MAP Human Cytokine Magnetic Bead Assay (Luminex MAP technology). Angiogenic biomarkers will be tested in aliquots of the serum samples at the following time points: baseline, 3-4 weeks, 3 and 12 months of curcumin consumption. Tissue biomarker (FGF-2, NF-kappaB p65, COX-2, pSTAT3) will be evaluated by IHC analysis and biomarker expression will be assessed by quantitative ACIS (Automated Cellular Imaging System, ACIS, Chromavision, Inc.). ACIS quantitation provides an objective precise analysis of biomarker expression and showed strong correlation with the study pathologist scoring results in our previous studies. IKKbeta kinase activity in salivary cells will be determined by a kit from Cell Signaling (Slaughter 1953).

It will also be determined if the levels of curcumin in serum correlate with curcumin-modulated effects on biomarkers implicated in the angiogenic switch and cell invasion. Spearman rank correlation analysis will be applied to determine if the levels of curcumin in serum correlate with curcumin-modulated effects on the biomarkers at the following time points: baseline, 3-4 weeks, 3 and 12 months of curcumin consumption. Serum and tissue biomarkers we propose to evaluate in this study were previously modulated by curcumin.

### References:

Aggarwal BB, et al. Targeting inflammatory pathways for prevention and therapy of cancer: short-term friend, long-term foe. Clin Cancer Res 2009;15:425-30.
Anand, P. Bioavailability of Curcumin: Problems and Promises. Mol Pharmaceutics 2007;4:807-18.
Anand P, et al. Cancer is a preventable disease that requires major lifestyle changes. Pharm Res 2008;25:2097-116.
Bisht S, Maitra A. Systemic delivery of curcumin: 21st century solutions for an ancient conundrum. Current Drug Discoveries Technology. 2009; 6(3):192-9.
Dhillon, N. Phase II Trial of Curcumin in Patients with Advanced Pancreatic Cancer. Clin Cancer Res., 2008;14:4491-9.
Franklin. Expression of elF4E During Head and Neck Tumorigenesis: Possible Role in Angiogenesis. The Laryngoscope. 109(8):1253-8
Gescher AJ, et al. Cancer chemoprevention by dietary constituents: a tale of failure and promise. The Lancet Oncol 2001;2:371-9.
Garcea G, et al. Detection of curcumin and its metabolites in hepatic tissue and portal blood of patients following oral administration. Br J Cancer, 2004;90:1011-15.
Garcea G, et al. Consumption of the putative chemopreventative agent curcumin by cancer patients: assessment of curcumin levels in the colorectum and their pharmacodynamics consequences. Cancer Epidemiol Biomarkers Prev 2005;14:120-25.
Hong, W., et al., Prevention of second primary tumors with isotretinoin in squamous-cell carcinoma of the head and neck. New England Journal of Medicine, 1990. 323(12): p. 795-801.
Ireson CR, et al. Metabolism of the cancer chemopreventative agent curcumin in human and rat intestine. Cancer Epidemiol. Biomarkers Prev 2002;11:105-11.
Lao et al., Dose escalation of a curcuminoid formulation. BMC Complement Altern Med 2006;6:10.
Lippman, S, et al, Cancer chemoprevention. Journal of Clinical Oncology, 1994. 12(4): p. 851-73.
Sharma RA, et al. Pharmacodynamic and pharmacokinetic study of oral curcuma extract in patients with colorectal cancer. Clin Cancer Res., 2001;7:1894-1900.
Sharma RA, et al. Phase I clinical trial of oral curcumin: biomarkers of systemic activity and compliance. Clin Cancer Res., 2004;10:6847-54.
Sharma RA, et al. Curcumin: the story so far. Eur J of Cancer 2005;41:1955-68
Slaughter DP, et al. "Field cancerization" in oral stratified squamous epithelium. Cancer 1953;6:963-68.
Vareed, S. K. Pharmacokinetics of Curcumin Conjugate Metabolites in Healthy Human Subjects. Cancer Epidemiol Biomarkers Prev, 2008;17:1411-7.

## Claims

1. A pharmaceutical composition for oral transmucosal delivery, comprising microsized curcumin, and a buffer to maintain the pH at 4.5-6.5, wherein the pharmaceutical composition is formed as a lozenge or a gum, and the microsized curcumin are between 100 and 5000 micrometers in size.

2. The pharmaceutical composition of claim 1, wherein said microsized curcumin is microgranular curcumin of between 500 and 1500 microns in size.

3. The pharmaceutical composition of claim 1, wherein said microsized curcumin is sterile.

4. The pharmaceutical composition of claim 1 further comprising nicotine.

5. A therapeutic amount of a composition of claim 1, wherein the amount of said composition is sufficient to provide a serum concentration of curcumin of at least 20 ng/mL for a period of from 1-2 hours following the initial administration for use in a method for providing a therapeutic blood serum concentration of curcumin in a patient, wherein said method involves oral transmucosal administration.

6. A composition of claim 1 for use in treating or preventing a tumor in a patient in need thereof by oral transmucosal administration.

7. The composition for use of claim 6, wherein the tumor is oral.

8. The composition for use of claim 6, wherein the tumor is non-oral.

## Patentansprüche

1. Pharmazeutische Zusammensetzung zur oralen transmucosalen Verabreichung, umfassend mikrosiertes Curcumin, und einen Puffer, um den pH-Wert bei 4,5-6,5 zu halten, wobei die pharmazeutische Zusammensetzung als eine Pastille oder ein Kaugummi ausgebildet ist, und das mikrosierte Curcumin eine Größe zwischen 100 und 5000 Mikrometer hat.

2. Pharmazeutische Zusammensetzung nach Anspruch 1, wobei das mikrosierte Curcumin mikrogranuläres Curcumin von einer Größe zwischen 500 und 1500 Mikronen ist.

3. Pharmazeutische Zusammensetzung nach Anspruch 1, wobei das mikrosierte Curcumin steril ist.

4. Pharmazeutische Zusammensetzung nach Anspruch 1, ferner umfassend Nikotin.

5. Therapeutische Menge einer Zusammensetzung nach Anspruch 1, wobei die Menge der Zusammensetzung ausreichend ist, um eine Serumkonzentation von Curcumin von zumindest 20 ng/mL über einen Zeitraum von 1-2 Stunden bereitzustellen, nach der ersten Verabreichung zur Verwendung in einem Verfahren zum Bereitstellen einer therapeutischen Blutserumkonzentation von Curcumin in einem Patienten, wobei das Verfahren orale transmucosale Verabreichung beinhaltet.

6. Zusammensetzung nach Anspruch 1 zur Verwendung bei der Behandlung oder Vorbeugung eines Tumors in einem behandlungsbedürftigen Patienten durch orale transmucosaler Verabreichung.

7. Zusammensetzung zur Verwendung nach Anspruch 6, wobei der Tumor oral ist.

8. Zusammensetzung zur Verwendung nach Anspruch 6, wobei der Tumor nicht oral ist.

## Revendications

1. Composition pharmaceutique destinée à une délivrance par voie transmuqueuse buccale, comprenant de la curcumine de taille micrométrique, et un tampon pour maintenir le pH de 4,5 à 6,5, dans laquelle la composition pharmaceutique est formée comme une pastille ou une gomme, et la curcumine de taille micrométrique mesure entre 100 et 5 000 micromètres de taille.

2. Composition pharmaceutique selon la revendication 1, dans laquelle ladite curcumine de taille micrométrique est de la curcumine microgranulaire mesurant entre 500 et 1 500 microns de taille.

3. Composition pharmaceutique selon la revendication 1, dans laquelle ladite curcumine de taille micrométrique est stérile.

4. Composition pharmaceutique selon la revendication 1 comprenant en outre de la nicotine.

5. Quantité thérapeutique d'une composition selon la revendication 1, dans laquelle la quantité de ladite composition est suffisante pour fournir une concentration sérique de curcumine d'au moins 20 ng/mL pendant une période de 1 à 2 heures suite à l'administration initiale destinée à être utilisée dans un procédé destiné à fournir une concentration sérique sanguine thérapeutique de curcumine chez un patient, dans laquelle ledit procédé implique une administration par voie transmuqueuse buccale.

6. Composition selon la revendication 1 destinée à être utilisée dans le traitement ou la prévention d'une tumeur chez un patient qui en a besoin par administration par voie transmuqueuse buccale.

7. Composition destinée à être utilisée selon la revendication 6, dans laquelle la tumeur est buccale.

8. Composition destinée à être utilisée selon la revendication 6, dans laquelle la tumeur est non buccale.
